# EUROPÄISCHE PATENTSCHRIFT

(11) **EP 2 136 921 B1**
(45) Veröffentlichungstag und Bekanntmachung des Hinweises auf die Patenterteilung: **29.08.2012**
(21) Anmeldenummer: 08735283.7
(22) Anmeldetag: 16.04.2008
(51) Int. Cl.: B01L 3/00, B01L 1/02, B01F 13/00, G01N 35/00, C12M 1/00

(54) **VERFAHREN UND VORRICHTUNG ZUR TROPFENMANIPULATION**
METHOD AND DEVICE FOR DROP MANIPULATION
PROCÉDÉ ET DISPOSITIF DE MANIPULATION DE GOUTTES

(30) Priorität: 17.04.2007 DE 102007018056
(43) Veröffentlichungstag der Anmeldung: 30.12.2009
(73) Patentinhaber: Fraunhofer-Gesellschaft zur Förderung der angewandten Forschung e.V., 80686 München (DE); Institut Für Bioprozess- und Analysenmesstechnik e.V., 37308 Heilbad Heiligenstadt (DE)
(72) Erfinder: ZIMMERMANN, Heiko, 60596 Frankfurt (DE); FUHR, Günter, 13187 Berlin (DE); GASTROCK, Gunter, 37308 Heilbad Heiligenstadt (DE); METZE, Josef, 37308 Heilbad Heiligenstadt (DE)
(74) Vertreter: Hertz, Oliver
(86) Internationale Anmeldenummer: PCT/EP2008/003041
(87) Internationale Veröffentlichungsnummer: WO 2008/125347

(56) Entgegenhaltungen:
- EP-A- 1 386 657
- EP-A- 1 707 938
- WO-A-2006/026351
- WO-A-2008/007511
- US-A1- 2003 183 525
- US-A1- 2007 242 105

## Beschreibung

Die Erfindung betrifft ein Verfahren zur Manipulation von flüssigen Tropfen, insbesondere von Tropfen mit biologischen Proben, unter der Wirkung elektrischer Felder und ein Kultivierungsgerät für biologische Zellen, das eine Vorrichtung zur Durchführung eines derartigen Verfahrens enthält.

Es ist bekannt, flüssige Tropfen auf einer Substratoberfläche durch die Wirkung eines elektrischen Feldes zu bewegen (siehe z. B. J. A. Schwartz et al. in "Lab on a Chip", Bd. 4, 2004, S. 11 - 17). Das elektrische Feld wird mit Elektroden erzeugt, die in die Substratoberfläche integriert sind. Es können ein lokal selektiv eingestelltes Gleichspannungsfeld, mit dem die Benetzung der Substratoberfläche gesteuert wird (so genanntes Elektrowetting-Verfahren, Elektrokapillarität), oder ein hochfrequentes elektrisches Feld erzeugt werden, mit dem der Tropfen durch negative oder positive Dielektrophorese oder Wanderwellen bewegt wird.

Beim Elektrowetting-Verfahren wird die Benetzung an entgegengesetzten Tropfenseiten verschieden eingestellt, so dass sich der Tropfen zu der Seite mit der stärkeren Benetzung verschiebt. Bei Anwendung eines hochfrequenten elektrischen Feldes bewirkt eine Polarisation des Tropfens eine Veränderung der Adhäsionskraft zwischen dem Tropfen und der Substratoberfläche. Die hochfrequenten elektrischen Felder können, zum Beispiel durch die Erzeugung von Wanderwellen an der Substratoberfläche, so erzeugt werden, dass die Veränderung der Adhäsionskraft auf einander entgegengesetzten Seiten des Tropfens verschieden stark ist. Im Ergebnis wird eine resultierende Kraft erzeugt, unter deren Wirkung sich der Tropfen entlang der Substratoberfläche bewegt.

Die praktische Anwendung der genannten Techniken unterliegt, wenn Tropfen mit biologischen Proben, insbesondere Zellen bewegt werden sollen, aufgrund der folgenden Probleme starken Beschränkungen. Erstens wurde festgestellt, dass Zellen in elektrisch bewegten Tropfen nicht-reproduzierbare Veränderungen, wie zum Beispiel eine verminderte Vitalität zeigen. Des Weiteren kann bei den herkömmlichen Techniken eine unerwünschte Deformation der Tropfen, zum Beispiel durch ein Breitfließen auftreten. Deformationen können jedoch von Nachteil sein, da diese mit unerwünschten Änderungen der geometrischen Anordnung der Zellen im Tropfen verbunden sind. Weitere Nachteile ergeben sich durch die beschränkte Kompatibilität mit anderen Techniken zum Umgang mit biologischen Proben, wie zum Beispiel einer Langzeitkultivierung, einer Ablage von Tropfen in Kompartimenten von Mikrotiterplatten oder einer Einstellung vorbestimmter Umgebungsbedingungen zur Aufrechterhaltung der Vitalität der Zellen.

Weitere Vorrichtungen zur Manipulation von flüssigen Tropfen sind aus EP-A-1 386 657, EP-A-1 707 938, WO 2006/026351 A, US 2007/242105 A1 und WO 2008/007511 A bekannt.

Die Aufgabe der Erfindung ist es, ein verbessertes Verfahren zur Manipulation flüssiger Tropfen bereitzustellen, mit dem die Nachteile der herkömmlichen Technik überwunden werden. Des weiteren ist es die Aufgabe der Erfindung, eine verbesserte Tropfenmanipulationsvorrichtung bereitzustellen.

Diese Aufgabe wird durch ein Verfahren oder eine Vorrichtung mit den Merkmalen der nebengeordneten Ansprüche gelöst. Vorteilhafte Ausführungsformen und Anwendungen der Erfindung ergeben sich aus den abhängigen Ansprüchen.

Gemäß einem ersten Gesichtspunkt wird die Aufgabe insbesondere durch die technische Lehre gelöst, mindestens einen Tropfen an einer Haltefläche einer Tropfenhalterung unter der Wirkung eines elektrischen Feldes zu manipulieren, wobei der Tropfen an der Haltefläche hängt. Der Tropfen ist mit der Haltefläche, an der das elektrische Feld erzeugt wird (oder am stärksten ist), ausschließlich durch eine Adhäsionskraft verbunden. Der Tropfen berührt die Haltefläche an einem oberen oder seitlichen Teil des Tropfens.

Der Tropfen enthält einen vorbestimmten Sammelbereich. Wenn sich im Tropfen mindestens ein suspendierter Partikel, insbesondere mindestens eine suspendierte biologische Zelle befindet, so sammeln sich diese unter der Wirkung der Gravitation im Sammelbereich. Das elektrische Feld wird an der Haltefläche erzeugt. Die größte Feldstärke wirkt auf den Tropfen in einem Kontaktbereich, in dem der Tropfen die Haltefläche berührt. Durch die Ausrichtung der Haltefläche derart, dass die Oberflächennormale keine Vektorkomponente entgegengesetzt zur Gravitationsrichtung aufweist, haben der Sammelbereich und der Kontaktbereich einen gegenseitigen Abstand. Der Sammelbereich ist an einem unteren Teil des Tropfens (Unterseite des Tropfens) gebildet. An der Unterseite, die in Richtung der Gravitationskraft weist, ist die Feldstärke des elektrischen Feldes im Vergleich zum Kontaktbereich vermindert. Suspendierte Partikel befinden sich im Tropfen in einem Bereich, in dem Kräfte, die durch das elektrische Feld induziert sind, vermindert sind oder der frei von derartigen Kräften ist.

Vorteilhafterweise können durch die hängende Anordnung des Tropfens an der Haltefläche suspendierte biologische Zellen in dem Sammelbereich angeordnet werden, dessen geometrischer Schwerpunkt vom Kontaktbereich getrennt ist, in dem die Kraft auf den Tropfen wirkt. Die Erfinder haben festgestellt, dass bei Einhaltung eines Abstandes der suspendierten Zellen vom Kontaktbereich die Belastung der Zellen durch das elektrische Feld vermindert wird. Unerwünschte Zellveränderungen werden vermieden. Die Einstellung vorbestimmter Kultivierungsbedingungen und ein schonender Umgang mit den Zellen werden vereinfacht.

Vorrichtungsbezogen wird die Aufgabe insbesondere durch die technische Lehre gelöst, ein Kultivierungsgerät für biologische Zellen, umfassend eine Tropfenmanipulationsvorrichtung mit einer Tropfenhalterung bereitzustellen, deren Haltefläche zur Aufnahme mindestens eines Tropfens eingerichtet ist und mit der Richtung der Erdanziehungskraft (Gravitation) einen Winkel kleiner oder gleich 90° bildet. Der an der Haltefläche durch eine Adhäsionskraft hängende Tropfen weist eine Form derart auf, dass sich suspendierte Zellen an der Unterseite des Tropfens sammeln können und damit einen Abstand von der Haltefläche aufweisen. Die Tropfenform wird vorteilhafterweise durch die Gravitationskraft bestimmt. Nicht-reproduzierbare Deformationen werden vermieden. Ein weiterer wichtiger Vorteil der Erfindung ist es, dass durch die hängende Anordnung die Bewegung des Tropfens erleichtert wird. Ein Breitfließen der Flüssigkeit wird vermieden, so dass der Kontaktbereich, in dem das elektrische Feld wirkt, kleiner als bei der herkömmlichen Bewegung von Tropfen auf Substraten ist.

Mit dem Begriff "hängender Tropfen" wird hier allgemein ein flüssiger Tropfen bezeichnet, der an einem oberen und/oder seitlichen Teil die Haltefläche berührt, an der das elektrische Feld zur Krafterzeugung gebildet wird und die insbesondere mit einer Elektrodeneinrichtung zur Erzeugung des elektrischen Feldes ausgestattet ist. Ein unterer Teil des hängenden Tropfens ist vorzugsweise frei, kann jedoch alternativ durch eine zusätzliche Bodenfläche eines Bodenelements unterstützt werden. Mit dem Begriff "elektrisches Feld" wird hier jedes elektromagnetische Feld oder jede Überlagerung von Feldern bezeichnet, das oder die zur Kraftausübung mit einem der bekannten Verfahren, insbesondere mit dem Elektrowetting-Verfahren, elektrischen Wanderwellen und/oder negativer oder positiver Dielektrophorese geeignet ist. Das elektrische Feld ist ein Gleichspannungsfeld oder ein Wechselspannungsfeld, das zur Kraftausübung entlang der Haltefläche lokal variiert wird. Mit dem Begriff "Manipulation" des Tropfens wird jede gezielte Beeinflussung (Kontrolle) der Position, Form, Größe und/oder Zusammensetzung des Tropfens an der Haltefläche bezeichnet.

Mit dem Begriff "Tropfenhalterung" wird jede Struktur bezeichnet, die eine nach unten und/oder zur Seite weisende feste Haltefläche aufweist, an der Tropfen anhaften können. Erfindungsgemäß ist die Haltefläche in einem Kultivierungsgerät vorgesehen. Die "Haltefläche" zur Aufnahme des Tropfens stellt allgemein eine feste Oberfläche dar, die vorzugsweise aus einem biologisch inerten Material, z. B. PTFE oder Glas besteht. Die Form der Haltefläche kann in Abhängigkeit von der konkreten Anwendung der Erfindung gewählt werden. Die Haltefläche kann insbesondere eine vollständig ebene Oberfläche, eine aus ebenen Teilbereichen verschiedener Neigung zusammengesetzte Oberflächen oder eine gekrümmte Oberfläche aufweisen. Die Oberfläche kann flächig oder streifenförmig gebildet sein. Die Haltefläche kann eine glatte oder eine strukturierte Oberfläche aufweisen.

Die Elektrodeneinrichtung zur Erzeugung des elektrischen Feldes umfasst fixierte Elektroden, die auf der Haltefläche angeordnet oder in diese eingebettet sind, oder alternativ mindestens eine bewegliche Elektrode, die auf einer Rückseite der Haltefläche verschiebbar angeordnet ist. Die Elektroden sind mit einer Steuereinrichtung verbunden und dazu eingerichtet, das elektrische Feld an der Haltefläche lokal zeitlich veränderlich zu bilden, so dass auf den Tropfen eine gerichtete Kraft ausgeübt wird.

Die Vorteile der Erfindung sind bei der Manipulation von Tropfen besonders ausgeprägt, die Partikel enthalten, die gegen das elektrische Feld empfindlich sind. Die Hauptanwendung ist mit Partikeln gegeben, die biologische Zellen umfassen. Die Zusammensetzung des erfindungsgemäß manipulierten Tropfens kann vorteilhafterweise in Abhängigkeit von der konkreten Aufgabenstellung gewählt werden. Gemäß einer ersten Variante kann der Tropfen mindestens eine suspendierte biologische Zelle enthalten. Der Tropfen mit der mindestens einen Zelle wird an der Haltefläche bewegt und optional Kultivierungsschritten, Behandlungen und/oder Messungen unterzogen. Gemäß einer zweiten Variante kann der Tropfen frei von Zellen sein. In diesem Fall besteht der Tropfen ausschließlich aus einer zellfreien Flüssigkeit, die zum Beispiel eine Nährlösung oder eine Flüssigkeit mit biologisch wirksamen Bestandteilen (einschließlich biologische Makromoleküle oder Zellbestandteile) umfasst. Bei der zweiten Variante umfasst die Manipulation des Tropfens (mit oder ohne mindestens eine Zelle) vorzugsweise eine Bewegung hin zu einem weiteren Tropfen (mit oder ohne mindestens eine Zelle), der an der Haltefläche angeordnet ist, wobei der weitere Tropfen mit der mindestens einen Zelle frei von elektrischen Feldern an der Haltefläche hängen kann oder ebenfalls der erfindungsgemäßen Manipulation unterzogen wird. In vorteilhafter Weise kann somit allgemein an der Haltefläche mindestens ein Tropfen mit mindestens einer Zelle bewegt und/oder mindestens ein zellfreier Tropfen zu einem zellfreien Tropfen oder zu einem Tropfen mit mindestens einer Zelle bewegt werden.

Gemäß bevorzugten Ausführungsformen der Erfindung können an der Haltefläche die folgenden Tropfenbewegungen einzeln oder in Kombination vorgesehen sein. Erstens können zwei oder mehr Tropfen miteinander verbunden werden, wobei mindestens einer der Tropfen mindestens eine Zelle enthält. Vorteilhafterweise können die verbundenen Tropfen sich gegenseitig beeinflussen, um vorbestimmte Bedingungen für die mindestens eine Zelle einzustellen. Die Verbindung von mindestens zwei Tropfen kann zum Beispiel eine Fusion von Tropfen umfassen, um einem ersten Tropfen mit mindestens einer Zelle eine Flüssigkeit, eine Wirksubstanz und/oder eine weitere Zelle zuzuführen. Alternativ kann die Verbindung von mindestens zwei Tropfen eine gegenseitige Anlagerung von Tropfen ohne eine gegenseitige Durchmischung umfassen. In diesem Fall wird vorteilhafterweise die Bildung gekrümmter Grenzflächen zwischen den Tropfen erreicht, die vorbestimmte Kultivierungsbedingungen für die mindestens eine Zelle bereitstellen. Des Weiteren können an Tropfen ohne eine gegenseitige Durchmischung vorteilhafterweise Substanzgradienten, insbesondere zur Einstellung von Kultivierungsbedingungen erzeugt werden.

Zweitens kann eine Bewegung des Tropfens entlang verschieden stark geneigten Teilbereichen der Haltefläche vorgesehen sein. Vorteilhafterweise kann in diesem Fall die Tropfenbewegung zusätzlich zu der Kraft durch das elektrische Feld auch durch die Neigung der Haltefläche gezielt beeinflusst werden.

Drittens kann die Bewegung des Tropfens zu einer Abtropfeinrichtung der Haltefläche führen, an der eine Trennung des Tropfens von der Haltefläche (Abtropfen) vorgesehen ist.

Viertens kann die Bewegung zu einer Stufenstruktur der Haltefläche (zum Beispiel Vorsprung oder Vertiefung) vorgesehen sein, an der vorteilhafterweise eine Fixierung des Tropfens durch eine erhöhte Adhäsionskraft vorgesehen ist.

Schließlich kann die Bewegung des Tropfens eine Verschiebung in einen feldfreien Teilbereich der Haltefläche umfassen, an dem vorteilhafterweise eine von elektrischen Feldern unbeeinflusste Kultivierung sichergestellt werden kann.

Vorteilhafte Varianten der Erfindung umfassen eine Verbindung von Tropfen, um vorbestimmte Tropfenmanipulationen auszuführen und/oder Kultivierungsbedingungen einzustellen. Beispielsweise stellt die Verbindung von Tropfen ein besonders einfaches Mittel zur Tropfenvergrößerung dar. Es können zu einem betrachteten Tropfen ein oder mehrere Tropfen zugeführt werden, bis eine kritische Abtropfdimension erreicht wird und der verbundene Tropfen aufgrund seines Eigengewichts von der Haltefläche abfällt. Vorteilhafterweise kann dieser Abtropfvorgang erfindungsgemäß gezielt gesteuert werden. Alternativ kann die Verbindung von Tropfen einer Kompensation eines Flüssigkeitsverlusts durch Verdunstung dienen. Gemäß einer weiteren Alternative umfasst die Verbindung von Tropfen eine Brückenbildung zwischen einander gegenüberstehenden vertikalen Teilbereichen der Haltefläche. Die Teilbereiche der Haltefläche bilden einen vertikal ausgerichteten Spalt, in dem mindestens zwei Tropfen unter der Wirkung von Adhäsionskräften einen verbundenen Tropfen mit einem vergrößerten Volumen bilden, das die kritische Abtropfdimension überschreiten kann.

Eine wichtige Anwendung der Verbindung von Tropfen besteht in der gezielten gegenseitigen Wechselwirkung der Tropfen. Es wird vorzugsweise mindestens ein Probentropfen mit mindestens einer Zelle und mindestens ein Substanztropfen mit mindestens einer Wirksubstanz zur Wechselwirkung gebracht, um die Zelle einer chemischen oder biologischen Behandlung zu unterziehen. Diese Ausführungsform der Erfindung ist insbesondere für die Kultivierung biologischer Zellen von Vorteil. Mit der Erfindung kann die Anwendung der herkömmlichen Kultivierung in hängenden Tropfen erheblich erweitert werden.

Die Haltefläche ist allgemein eine geschlossene Oberfläche, die zusätzlich zu den optional vorgesehenen Komponenten, wie zum Beispiel der Abtropfeinrichtung, dem feldfreien Teilbereich und/oder der Stufenstruktur des Weiteren mit mindestens einer Durchgangsöffnung ausgestattet sein kann. Die Durchgangsöffnung ist eine Durchbrechung der Haltefläche. Die Dimension der Durchgangsöffnung ist vorzugsweise so gewählt, dass ein Tropfen unter der Wirkung der Adhäsionskraft in der Durchgangsöffnung aufgespannt hängen kann. Die Dimension der Durchgangsöffnung ist z. B. im Bereich von 50 µm bis 10 mm, insbesondere von 100 µm bis 5 mm gewählt. Alternativ kann die Durchgangsöffnung einen größeren Durchmesser aufweisen, so dass Tropfen am Umfangsrand der Durchgangsöffnung adhärent hängen können.

Vorteilhafterweise wird mit der Durchgangsöffnung die Funktion der Haltefläche und damit die Anwendung der Erfindung erheblich erweitert. An der Durchgangsöffnung kann eine Tropfenzufuhr zu der Haltefläche, eine Fixierung von mindestens einem Tropfen, eine Spülung des Tropfens mit einer Perfusionseinrichtung, eine weitere Tropfenzufuhr und/oder eine Abnahme des Tropfens von der Haltefläche vorgesehen sein, wobei vorteilhafterweise an sich bekannte Werkzeuge der Zellbiologie, wie zum Beispiel eine Pipettiereinrichtung, eine Perfusionseinrichtung und/oder eine Tropfenzufuhreinrichtung verwendbar sind. Die Durchgangsöffnung stellt eine Schnittstelle zur Verbindung der Tropfenmanipulation an der Haltefläche mit weiteren zellbiologischen Verfahrensschritten dar.

Wenn gemäß einer weiteren Ausführungsform der Erfindung eine Messung an dem Tropfen an der Haltefläche vorgesehen ist, kann vorteilhafterweise der Zustand von dem Tropfen oder insbesondere einer im Tropfen enthaltenen Zelle überwacht werden. Die Messung eines an der Durchgangsöffnung angeordneten Tropfens ist bevorzugt, da in diesem Fall herkömmliche Methoden der optischen Beobachtung, wie zum Beispiel eine mikroskopische Beobachtung anwendbar ist. Alternativ kann eine elektrische Messung, insbesondere unter Verwendung der Elektrodeneinrichtung an der Haltefläche vorgesehen sein.

Wichtige Vorteile der Erfindung können wie folgt zusammengefasst werden. Die Gravitation zieht suspendierte Zellen in Tropfen auf eine Tropfengrenzfläche, die von Elektroden zur Erzeugung des elektrischen Feldes zur Tropfenmanipulation abgewandt ist. Somit wird die elektrische Belastung im elektromagnetischen Feld gegenüber den herkömmlichen Techniken erheblich verringert. Die Kultivierung von Zellen in hängenden Tropfen wird vereinfacht. Mit der Anordnung der Zellen an der Tropfengrenzfläche ist vorteilhafterweise im Vergleich zu der herkömmlichen Tropfenmanipulation die Weglänge zwischen den Zellen und der Atmosphäre verkürzt. Somit ergibt sich eine verbesserte Gasversorgung der Zellen selbst (z. B. mit Sauerstoff) oder der Umgebung der Zellen im Tropfen (z. B. mit Kohlendioxid zur pH-Steuerung). Des Weiteren können Flüssigkeiten besonders einfach in einen Tropfen mit Zellen eingeführt werden, z. B. um eine Verdunstung zu kompensieren. Die Erfindung eignet sich somit insbesondere gut für eine Langzeitkultivierung über einen Zeitraum von Tagen oder Wochen. Das erfindungsgemäß induzierte Abtropfen der Tropfen von der Haltefläche, zum Beispiel durch die Bewegung zu einer Abtropfeinrichtung oder durch die Verbindung von Tropfen ermöglicht eine einfache und definierte Ablage von Tropfen mit oder ohne Zellen. Schließlich ermöglicht die Erfindung eine einfache Zuführung von Medien oder Zellen oder anderen in flüssigen Tropfen suspendierten Komponenten zu einer Zellsuspension.

Weitere Einzelheiten und Vorteile der Erfindung werden im Folgenden unter Bezug auf die beigefügten Zeichnungen beschrieben. Es zeigen:

| | |
|---|---|
| Figuren 1A bis 1C: | verschiedene Varianten der erfindungsgemäßen Manipulation eines hängenden Tropfens; |
| Figur 2: | eine herkömmliche Manipulation eines auf seiner Unterseite unterstützten Tropfens (Stand der Technik); |
| Figur 3: | eine schematische Illustrationen einer Ausführungsform einer gemäß der Erfindung verwendeten Elektrodeneinrichtung; und |
| Figuren 4 bis 11, 13 bis 19, 24 und 25: | schematische Illustrationen verschiedener Ausführungsformen und Anwendungen der Erfindung. |
| Figuren 12 und 20 bis 23: | schematische Illustrationen verschiedener Tropfenmanipulationsvorrichtungen, die nicht erfindungsgemäß sind. |

Bei der Beschreibung von bevorzugten Merkmalen und Anwendungen der erfindungsgemäßen Manipulation hängender Tropfen wird auf schematische Darstellungen von Ausführungsformen der erfindungsgemäß verwendeten Tropfenmanipulationsvorrichtung, insbesondere mit der Haltefläche und der Elektrodeneinrichtung Bezug genommen. Dabei ist insbesondere die Ausrichtung der Haltefläche zur Bereitstellung hängender Tropfen illustriert. Weitere Einzelheiten der Tropfenmanipulationsvorrichtung, insbesondere in Bezug auf die Größe, Form, Anordnung und Ansteuerung von Elektroden werden nicht beschrieben, da diese an sich von den herkömmlichen Verfahren zur Bewegung von Tropfen auf Substratoberflächen bekannt sind.

Es wird beispielhaft auf Tropfen Bezug genommen, die mindestens eine suspendierte biologische Zelle enthalten. Einzelheiten der biochemischen Behandlung von Zellen, z. B. der Kultivierung oder Differenzierung insbesondere von Stammzellen oder undifferenzierten Vorläuferzellen werden nicht beschrieben, da diese dem Fachmann an sich bekannt sind. Alternativ können andere suspendierte Partikel im Tropfen enthalten sein, z. B. Kunststoffpartikel, die Träger von Substanzen, z. B. von biologischen Wirkstoffen sind, oder Verbundpartikel aus Kunststoffpartikeln und biologischen Zellen.

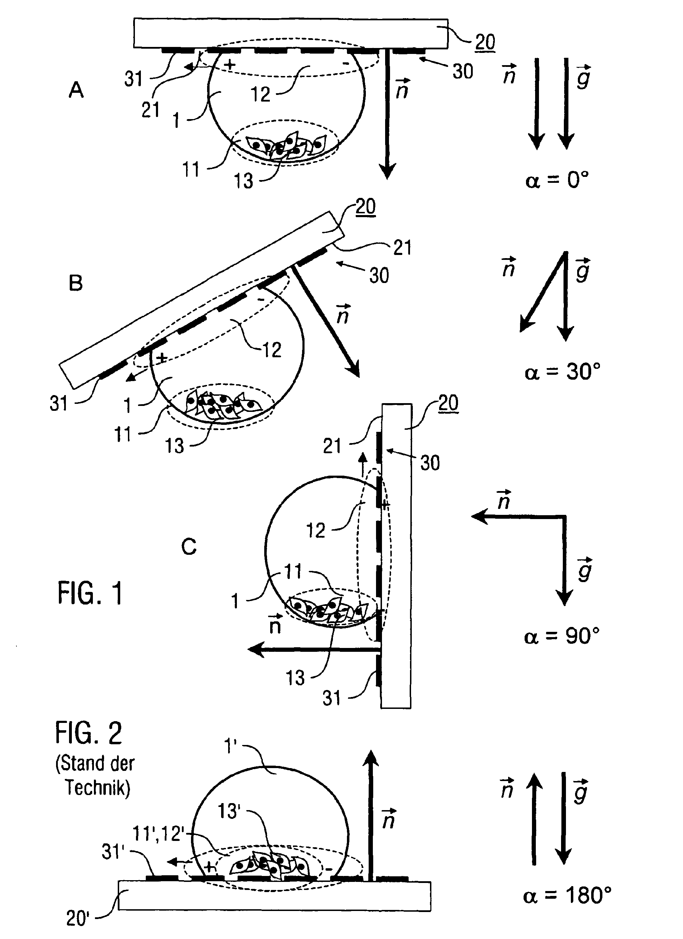

Die erfindungsgemäße Anordnung eines hängenden Tropfens 1 an einer vertikal nach unten oder horizontal zur Seite weisenden Haltefläche 21 einer Tropfenhalterung 20 ist gemäß Figur 1 im Unterschied zur herkömmlichen Auflage eines Tropfens 1' auf der Oberfläche eines Substrats 20' gemäß Figur 2 (Stand der Technik) gezeigt. Die Tropfenhalterung 20 ist Teil eines nicht dargestellten Kultivierungsgerätes für biologische Zellen. Sie hat z. B. die Form einer ebenen oder gekrümmten Platte. An der Tropfenhalterung 20 ist eine Elektrodeneinrichtung 30 vorgesehen, die fixierte Elektroden 31 auf der Haltefläche 21 umfasst. Die fixierten Elektroden 31 sind z. B. streifenförmig gebildet und senkrecht zur Zeichenebene angeordnet.

Zur Darstellung der Ausrichtung der erfindungsgemäß verwendeten Haltefläche 21 wird auf den Vektor der Oberflächennormalen (Vektor *n̅*) und die Richtung der Gravitation (Vektor *̅g̅*̅) Bezug genommen. Die positive Richtung der Gravitation (negative z-Richtung) weist zum Erdmittelpunkt vertikal nach unten. Die Haltefläche 21 kann vollständig eben sein oder eine aus ebenen Teilbereichen verschiedener Neigung zusammengesetzte Oberfläche oder eine gekrümmte Oberfläche aufweisen. Der Vektor der Oberflächennormalen steht an der betrachteten Position des Tropfens 1 senkrecht auf der Haltefläche 21. Die erfindungsgemäß verwendete Haltefläche 21 hat eine andere Ausrichtung als das herkömmlich verwendete Substrat, wie unter Bezug auf den Winkel α zwischen den Vektoren *̅n̅*̅ und *̅g̅*̅ gezeigt ist.

In den Figuren 1A bis 1C sind verschiedene Varianten der Erfindung gezeigt, bei denen der Winkel α im Bereich von 0° bis 90° gewählt ist. In jedem Fall sammeln sich die suspendierten Zellen 13 unter der Wirkung der Gravitation an einer Unterseite des Tropfens 1. Der Teil des Tropfens 1, in dem sich die Zellen 13 sammeln, wird als Sammelbereich 11 bezeichnet. Der Bereich des Tropfens 1, in dem das elektrische Feld der Elektrodeneinrichtung 30 wirkt, wird als Kontaktbereich 12 bezeichnet. Gemäß Figur 2 sind bei dem herkömmlich vertikal nach oben weisenden Substrat die Vektoren *̅n̅*̅ und *̅g̅*̅ zueinander entgegengesetzt ausgerichtet. Der Winkel zwischen beiden Vektoren beträgt 180°. In diesem Fall sind der Sammelbereich 11' und der Kontaktbereich 12' im Wesentlichen gleich. Entsprechend unterliegen die Zellen 13' im Sammelbereich 11' der Wirkung des elektrischen Feldes.

Im Unterschied zur herkömmlichen Technik sind bei der erfindungsgemäßen Technik gemäß Figur 1 die Sammel- und Kontaktbereiche 11, 12 voneinander getrennt. Eine Wirkung des elektrischen Feldes auf die Zellen 13 wird vorteilhafterweise vermieden. Die im Fall von Figur 1C gezeigte Überlappung ist in der Realität unkritisch, da sich der Sammelbereich 11 im Wesentlichen an der unteren Grenzfläche des Tropfens zur umgebenden Atmosphäre befindet.

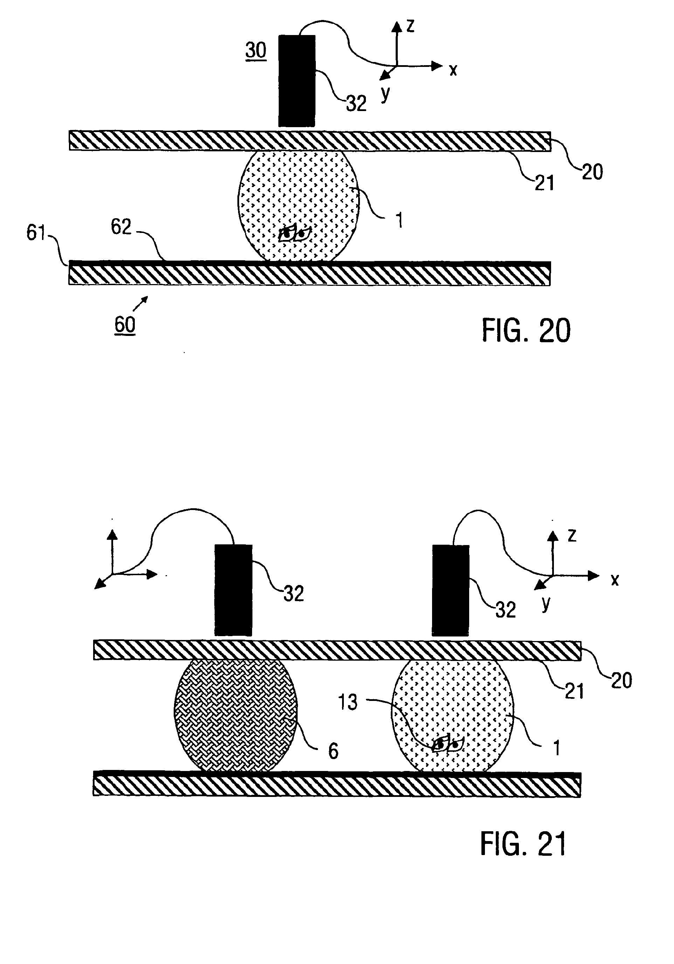

Die Elektrodeneinrichtung 30 ist allgemein zur Erzeugung eines örtlich veränderlichen elektrischen Feldes eingerichtet, unter dessen Wirkung eine Kraft auf den hängenden Tropfen 1 ausgeübt wird. Für die Erzeugung des lokal veränderlichen Feldes weist die Elektrodeneinrichtung 30 gemäß einer ersten gezeigten Variante eine Elektrodenanordnung mit einer Vielzahl einzeln ansteuerbarer fixierter Elektroden 31 auf (siehe Figuren 1 und 3 bis 19). Die fixierten Elektroden 31 sind streifenförmig nebeneinander angeordnet. Eine Bezugsrichtung senkrecht zur Ausrichtung der streifenförmigen Elektroden 31 bestimmt die Bewegungsrichtung der hängenden Tropfen 1. Gemäß einer zweiten Variante wird das lokal veränderliche elektrische Feld mit einer beweglichen Elektrode 32 erzeugt (Figuren 20 bis 25). Die bewegliche Elektrode 32 ist z. B. ein stabförmiges Element, das auf einer Seite (Oberseite) der Haltefläche 21 in mindestens einer Raumrichtung beweglich angeordnet ist. Bei Verwendung der beweglichen Elektrode 32 ist es vorteilhafterweise nicht erforderlich, dass die Haltefläche 21 mit integrierten Elektroden ausgestattet ist. Allerdings sind auch Ausführungsformen der Erfindung möglich, bei denen zur Tropfenmanipulation sowohl die fixierten Elektroden 31 auf oder in der Haltefläche 21 als auch die beweglichen Elektroden 32 vorgesehen sind.

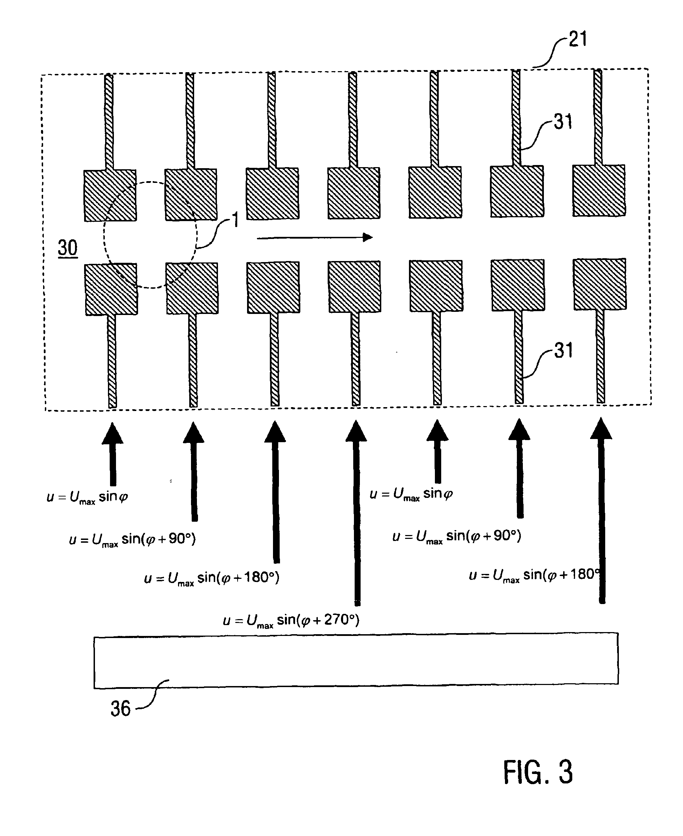

Figur 3 zeigt beispielhaft eine Draufsicht auf einen Ausschnitt einer Haltefläche 21 mit einer Elektrodeneinrichtung 30, die fixierte, streifenförmige Elektroden 31 umfasst. Es sind jeweils zwei fixierte Elektroden 31 paarweise angeordnet, an die mit einer schematisch gezeigten Spannungsversorgungseinrichtung 36 selektiv eine Wechselspannung (Frequenz z. B. 1 MHz) mit einer spezifischen Phasenverschiebung angelegt wird. Es bildet sich eine Wanderwelle, unter deren Wirkung der schematisch gezeigte Tropfen 1 senkrecht zur Ausrichtung der streifenförmigen Elektroden 31 (siehe Pfeil) bewegt wird.

Die Haltefläche 21 besteht z. B. aus Glas, auf das die fixierten Elektroden 31 z. B. aus Gold durch Sputtern aufgebracht sind. Die Elektroden 31 weisen an ihren Enden jeweils eine Erweiterung (sog. Pad) auf, in deren Bereich der Tropfen vorzugsweise angeordnet ist. Die Größe und Anordnung der Erweiterungen ist z. B. so gewählt, dass ein Tropfen mindestens zwei Pad's, z. B. vier oder sogar sechs Pad's überdeckt.

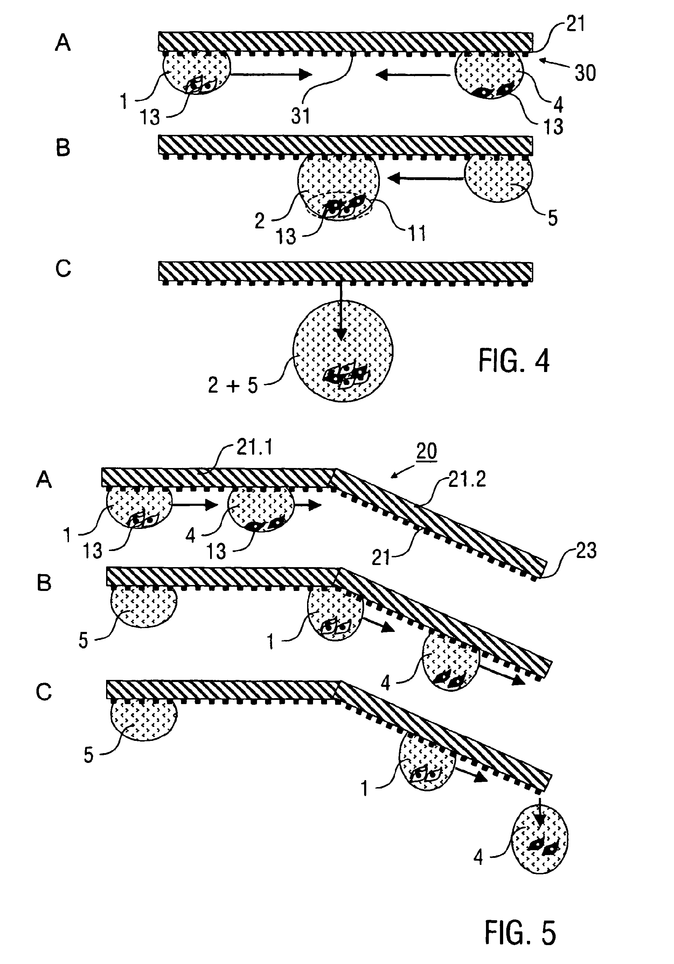

Die Figuren 4 bis 11, 13 bis 19, 24 und 25 illustrieren bevorzugte Verfahrensweisen, die mit der erfindungsgemäß verwendeten Tropfenmanipulationsvorrichtung realisierbar sind. Die gezeigten Ausführungsformen und Varianten können einzeln oder wiederholt oder in Kombination als Teilschritte eines zellbiologischen Verfahrens vorgesehen sein. Eine Aufgabe in der Zellbiologie ist z. B. die Bereitstellung einer Wechselwirkung von verschiedenen Zelltypen für Kultivierungszwecke. Beispielsweise umfasst ein erster Zelltyp Stammzellen oder undifferenzierte Vorläuferzellen, während der zweite Zelltyp differenzierte Zellen umfasst. Um eine gezielte Differenzierung der Stammzellen oder Vorläuferzellen zu induzieren, werden beide Zelltypen miteinander in Kontakt gebracht. Die Erfindung ermöglicht eine genaue und reproduzierbare Verbindung von Tropfen mit verschiedenen Zelltypen, wie in den Figuren 4 und 5 schematisch illustriert ist.

Gemäß Figur 4A sind Tropfen 1, 4 mit Zellen 13 verschiedener Zelltypen an der Haltefläche 21 mit der Elektrodeneinrichtung 30 hängend angeordnet. Die Elektrodeneinrichtung 30 umfasst gemäß der o. g. ersten Variante eine Vielzahl von geraden drahtförmigen Elektroden 31, die auf der Oberfläche der Haltefläche 21 angeordnet sind. Die streifenförmigen Elektroden 31 verlaufen senkrecht zur Zeichenebene der Figur 4 und relativ zueinander parallel. Bei Beaufschlagung der streifenförmigen Elektroden 31 mit hochfrequenten elektrischen Feldern werden Wanderwellen erzeugt. Die Wanderwellen verlaufen senkrecht zur Ausrichtung der streifenförmigen Elektroden 31.

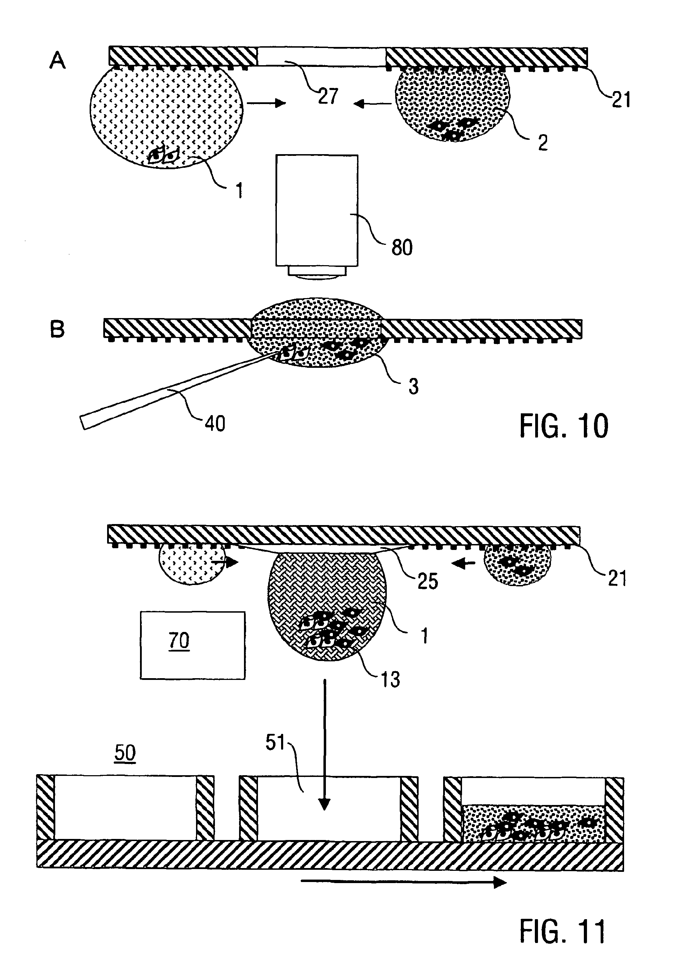

Die Elektroden 31 sind mit einer Steuereinrichtung (nicht dargestellt) verbunden, mit der jede der Elektroden 31 selektiv ansteuerbar sind. Beim dargestellten Beispiel werden in zwei Teilbereichen der Haltefläche 21 getrennt Wanderwellen mit entgegengesetzten Richtungen erzeugt, unter deren Wirkung die Tropfen 1, 4 zueinander bewegt werden (Figur 4A). Im Ergebnis wird ein verbundener Tropfen 2 gebildet (Figur 4B), der im Sammelbereich 11 Zellen 13 beider Zelltypen enthält. Zur Überführung des verbundenen Tropfens 2 wird im weiteren Verfahren ein zellfreier Tropfen 5 mit dem Tropfen 2 verbunden, so dass dieser unter der Wirkung seines Gewichts von der Haltefläche 21 abreißt (Figur 4C) und in eine Tropfenaufnahme fällt (in Figur 4 nicht dargestellt, siehe Figur 11).

Vorteilhafterweise befinden sich während des gesamten Verfahrens die Zelltypen in den getrennten Tropfen 1, 4 oder dem verbundenen Tropfen 2 aufgrund der nach unten gerichteten Gravitationskraft an der unteren Grenzfläche des Tropfens zur umgebenden Atmosphäre (Sammelbereich 11). Im Ergebnis werden die Zellen 13 vom elektrischen Feld der Elektroden 31 nicht beeinflusst.

Das Abtropfen durch eine Durchmesservergrößerung gemäß den Figuren 4B und 4C ist optional vorgesehen. Alternativ kann ein Abtropfen durch eine Änderung der Oberflächeneigenschaften der Haltefläche 21, zum Beispiel durch eine veränderte Ansteuerung der Elektroden 31 (mittel Elektrowetting) oder eine Bewegung des verbundenen Tropfens 2 in einen Teilbereich mit verminderter Adhäsion induziert werden. Gemäß einer weiteren Alternative kann ein Abtropfen von einer Abtropfkante 23 vorgesehen sein, wie schematisch in Figur 5 illustriert ist.

Bei der in Figur 5 gezeigten Ausführungsform der Erfindung umfasst die Haltefläche 21 einen ersten ebenen Teilbereich 21.1, dessen Oberflächennormale senkrecht nach unten weist (α = 0°), und einen zweiten ebenen Teilbereich 21.2 mit einer veränderten Neigung relativ zur Gravitationsrichtung. Die Tropfen 1, 4 mit Zellen 13 verschiedener Zelltypen werden unter der Wirkung von elektrischen Wanderwellen vom ersten Teilbereich 21 zum geneigten Teilbereich 21.2 verschoben (Figur 5A). Im zweiten Teilbereich 21.2 laufen die Tropfen unter der Wirkung der Gravitation oder aktiv durch Wanderwellen gefördert bis zum Ende des Teilbereichs 21.2, der die Abtropfkante 23 bildet (Figur 5B). Die Fusion beider Tropfen 1, 4 kann an der Abtropfkante 23 oder alternativ nach dem Abtropfen in einem Auffangreservoir (Figur 5C) erfolgen.

Das Abtropfen an der Abtropfkante 23 kann durch eine hydrophobe Beschichtung und/oder eine Strukturierung (zum Beispiel Spitze, Zapfen) der Abtropfkante 23 gefördert werden. Alternativ oder zusätzlich kann ein Abtropfen durch eine Vergrößerung des verbundenen Tropfens mit einem Zusatztropfen 5 über eine kritische Abtropfdimension hinaus ausgelöst werden.

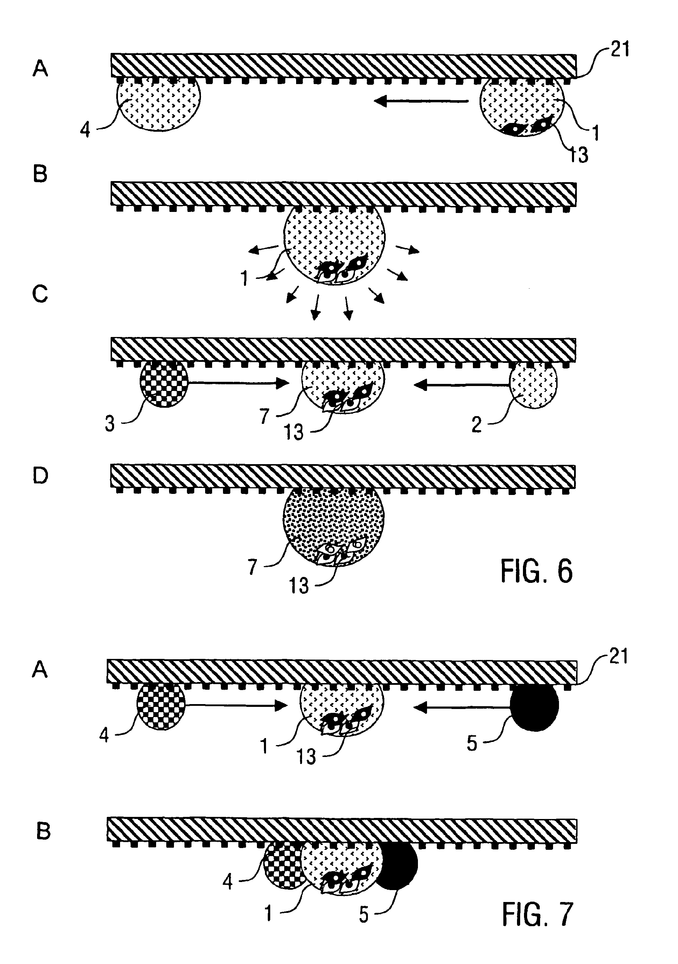

Die Figuren 6A bis 6D zeigen weitere Varianten der erfindungsgemäßen Tropfenmanipulation. Gemäß Figur 6A wird ein erster Tropfen 1 mit Zellen 13 zu einem zweiten, ruhenden Tropfen 4 ohne Zellen bewegt. Der Tropfen 4 enthält zum Beispiel eine Wirksubstanz, wie eine Nährstofflösung zur Versorgung der Zellen 13 oder einen Differenzierungsfaktor zur Auslösung einer Differenzierung der Zellen 13. Der aus den Tropfen 1 und 4 gebildete Kultivierungstropfen 7 (Figur 6B) kann zur Kultivierung, zum Beispiel zur Differenzierung von Zellen über eine vorbestimmte Kultivierungszeit, zum Beispiel Stunden, Tage oder Wochen im hängenden Zustand gehalten werden. Während der Kultivierung kann das elektrische Feld abgeschaltet werden. Bei der Kultivierung können die Zellen 13 im Tropfen 2 beispielsweise wachsen, sich vermehren, sich differenzieren, Aggregate bilden und/oder Gewebeformationen bilden, wie dies von der herkömmlichen Kultivierung in hängenden Tropfen bekannt ist.

Während der Kultivierung kann es zum Flüssigkeitsverlust durch Verdunstung kommen (siehe Pfeile in Figur 6B). Die Verdunstung stellt bei der herkömmlichen Kultivierung insbesondere in kleinen Tropfen (Durchmesser z. B. kleiner 0.5 mm) ein wesentliches Problem dar, da eine genaue Kompensation des Flüssigkeitsverlusts bisher schwer realisierbar war. Mit dem erfindungsgemäßen Verfahren kann der Flüssigkeitsverlust leicht kompensiert werden (Figur 6C) und insbesondere eine Kultivierung verlängert werden, indem zu dem Kultivierungstropfen 7 ein weiterer Tropfen 2 mit Wasser und/oder Nährstofflösung und/oder ein weiterer Tropfen 3 mit einer Wirksubstanz und/oder Zellen zugeführt wird.

Im Ergebnis kann der Kultivierungstropfen 7 für die gewünschte Kultivierungszeit im hängenden Zustand gehalten werden (Figur 6D), beispielsweise bis alle Zellen 13 differenziert sind. Anschließend kann beispielsweise das obengenannte Abtropfen in ein Auffangreservoir vorgesehen sein (nicht dargestellt).

Figur 7 zeigt eine weitere Ausführungsform der erfindungsgemäßen Tropfenmanipulation, bei der eine Verbindung von mehreren Tropfen eine gegenseitige Anlagerung ohne eine Durchmischung umfasst. Beispielsweise wird gemäß Figur 7A ein hängender Tropfen 1 mit suspendierten Zellen 13 an der Haltefläche 21 angeordnet. Die Zellen 13 umfassen zum Beispiel Stammzellen und differenzierte Zellen. Mit dem erfindungsgemäßen Verfahren wird mindestens ein weiterer Tropfen 4, 5 zu dem Tropfen 1 bewegt und an diesen angelagert. Eine Durchmischung der Tropfen wird verhindert, indem die Tropfen beispielsweise aus nicht-mischbaren Flüssigkeiten (Wasser/Öl) gebildet sind oder die Oberfläche des Tropfens 1 mit einem Film überzogen ist, der die Durchmischung unterbindet. Der Film besteht zum Beispiel aus Lipiden.

In Figur 7B ist beispielhaft die Anlagerung der zwei weiteren Tropfen 4, 5 auf verschiedenen Seiten des Tropfens 1 mit den Zellen 13 gezeigt. Die Tropfen 4, 5 enthalten eine Wirksubstanz mit verschiedenen Konzentrationen oder verschiedene Wirksubstanzen. Durch Diffusion der Wirksubstanz(en) kann ein Wirksubstanzgradient im Tropfen 1 gebildet werden, wie dies für in vitro-Kulturen oder die Techniken des Tissue-Engineering gewünscht wird. Des Weiteren werden zwischen den angelagerten, sich nicht durchmischenden Teiltropfen gekrümmte Grenzflächen gebildet, welche die Kultivierung der Zellen und insbesondere die Bildung von Gewebeformationen begünstigen können.

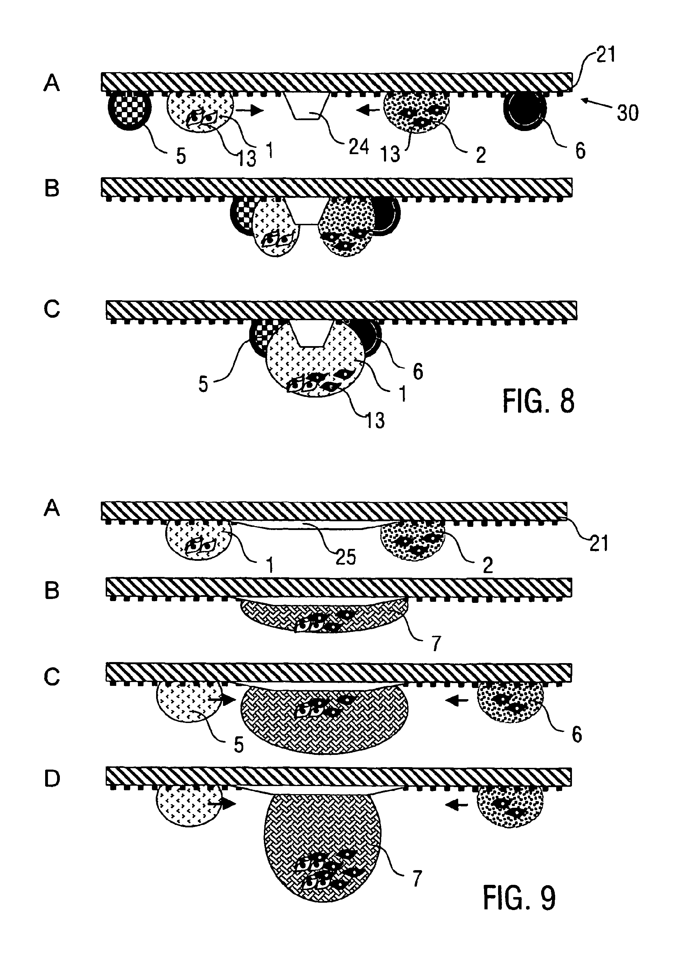

Die Haltefläche 21 der erfindungsgemäß verwendeten Tropfenmanipulationsvorrichtung kann mit einer Stufenstruktur 24 ausgestattet sein, die schematisch in Figur 8 illustriert ist. Die Stufenstruktur 24 umfasst zum Beispiel einen konusförmigen Vorsprung oder einen Steg mit einem trapezförmigen Querschnitt. Die Stufenstruktur 24 dient beispielsweise der Halterung eines Kultivierungstropfens 7 mit angelagerten Substanztropfen 5, 6.

Die Bildung der Zusammensetzung aus den Tropfen 5, 6 und 7 umfasst einen ersten Teilschritt (Figur 8A), bei dem von zwei verschiedenen Seiten zwei Probentropfen 1, 2, die Zellen 13, zum Beispiel Stammzellen und differenzierte Zellen enthalten, zu der Stufenstruktur 24 bewegt werden. Zusätzlich werden von den beiden Seiten die Wirksubstanztropfen 5, 6 an die Probentropfen 1, 2 angelagert. Vor Beginn der Differenzierung (Figur 8B) sind die Probentropfen 1, 2 mit den verschiedenen Zelltypen voneinander getrennt, so dass die Differenzierung noch nicht erfolgen kann und auch ein Gradient zwischen den Wirksubstanztropfen 5, 6 nicht ausgebildet werden kann. In einem weiteren Teilschritt (Figur 8C) werden die Teiltropfen zusammengeschoben, um den verbundenen Kultivierungstropfen 7 zu bilden, auf dessen entgegengesetzten Seiten die Wirksubstanztropfen 5, 6 angelagert sind. Die Wirksubstanztropfen 5, 6 sind durch die Stufenstruktur 24 voneinander getrennt und mit den Kultivierungstropfen 7 nicht mischbar (siehe oben).

Die Kultivierung an der Stufenstruktur 7 hat den Vorteil, dass die Oberfläche der Haltefläche 21, an welcher der Kultivierungstropfen 7 durch Adhäsionskräfte hängend gehalten wird, durch die Stufenstruktur 24 vergrößert wird. Im Ergebnis kann der Kultivierungstropfen 7 bis zu einem größeren Tropfendurchmesser gebildet und an der Haltefläche 21 gehalten werden als im Fall einer ebenen Fläche ohne die Stufenstruktur. Mit der Stufenstruktur 24 kann die kritische Abtropfdimension, oberhalb derer ein Abtropfen auftritt, vergrößert werden. Im vergrößerten Kultivierungstropfen 7 können größere Gewebeformationen kultiviert werden.

Die konusförmige Stufenstruktur 24 ist in Figur 8 als Beispiel gezeigt. Im Rahmen der Erfindung können zahlreiche Stufenstrukturen mit dieser oder einer abgewandelten Form, die von der Ebene der Haltefläche 21 vorstehen oder in dieser eine Ausnehmung bilden, vorgesehen sein. Mit der Stufenstruktur wird die Adhäsionsfläche des Kultivierungstropfens 7 vergrößert. Des Weiteren werden geometrische Bedingungen für die Anlagerung von Wirksubstanztropfen vorgegeben, wie dies insbesondere bei der Stammzellenkultivierung oder der Zellklonierung von Interesse ist.

Die Größe und Form der Stufenstruktur 24 kann vom Fachmann in Abhängigkeit von den konkreten Anforderungen bei der Anwendung der Erfindung gewählt werden. Die senkrechte Höhe oder Tiefe der Stufenstruktur 24 in Bezug auf die Ebene der Haltefläche 21 kann beispielsweise im Bereich von 50 µm bis 10 mm, insbesondere von 100 µm bis 5 mm gewählt sein.

Gemäß einer weiteren Variante der Erfindung kann die Haltefläche 21 mit mindestens einem feldfreien Teilbereich 25 ausgestattet sein, der schematisch in Figur 9 gezeigt ist. Der feldfreie Teilbereich 25 stellt einen flachen, zum Beispiel schichtförmigen Bereich der Haltefläche 21 dar, indem vorzugsweise keine Elektroden angeordnet sind. Der feldfreie Teilbereich 25 bildet ein Trennelement, auf das beidseitig Tropfen 1, 2 (Figur 9A) aufgeschoben werden können. Die laterale Breite des feldfreien Teilbereichs 25 ist vorzugsweise so gewählt, dass im aufgeschobenen Zustand die Tropfen 1, 2 zu einem verbundenen Kultivierungstropfen 7 fusionieren können (Figur 9B). Anschließend folgt die Zufuhr von weiterer Wirksubstanz und/oder Zellen mit den Tropfen 5, 6, die ebenfalls von den entgegengesetzten Seiten an den feldfreien Teilbereich 25 herangeführt werden, bis sie mit dem Kultivierungstropfen 7 fusionieren (Figur 9C). Schließlich kann nach einer vorbestimmten Kultivierungszeit der Kultivierungstropfen 7 durch Flüssigkeitszufuhr mit Teiltropfen so vergrößert werden, dass er die kritische Abtropfdimension überschreitet und in ein Auffangreservoir (nicht dargestellt) abtropft (Figur 9D).

Der feldfreie Teilbereich 25 hat den besonderen Vorteil, dass mit einer Oberflächenbeschichtung ein Breitfließen des Kultivierungstropfens 7 bereitgestellt werden kann. Es wird beispielsweise ein hydrophile Beschichtung vorgesehen, die eine Verteilung des Kultivierungstropfens 7 auf der gesamten Oberfläche des feldfreien Teilbereichs bewirkt. Mit der Beladung des feldfreien Teilbereichs 25 von einer oder mehreren Seiten können vorkultivierte Zellpopulationen gesteuert vereinigt und/oder kultiviert werden. Bevorzugte Anwendungen der in Figur 9 illustrierten Technik bestehen bei der Formung von Zellaggregaten, wie zum Beispiel von embryoiden oder organoiden Körpern bei der Differenzierung embryonaler oder adulter Stammzellen.

Zur Überwachung des mindestens einen Tropfens, der erfindungsgemäß an der Haltefläche 21 im hängenden Zustand gehalten und manipuliert wird, kann gemäß einer weiteren Variante der Erfindung eine Messung, wie zum Beispiel eine optische und/oder elektrische Messung vorgesehen sein. Beispielsweise kann am Kultivierungstropfen 7 eine Fluoreszenzmessung mit einem unterhalb der Haltefläche 21 angeordneten Mikroskop oder eine elektrische Impedanzmessung vorgesehen sein, für die vorzugsweise die Elektroden 31 der Elektrodeneinrichtung auf der Haltefläche 21 verwendet werden.

Die Messung am Tropfen kann vereinfacht werden, wenn in der Haltefläche 21 eine Durchgangsöffnung 27 vorgesehen ist (siehe Figur 10). Die Durchgangsöffnung 27 ist zum Beispiel ein rundes Loch oder ein Schlitz in dem Körper, der die Haltefläche 21 bildet. Die lichte Weite der Durchgangsöffnung 27 ist in Abhängigkeit von der konkreten Anwendung und den verwendeten Materialien so gewählt, dass der verbundene Tropfen 3 in der Durchgangsöffnung 27 sicher gehalten werden kann.

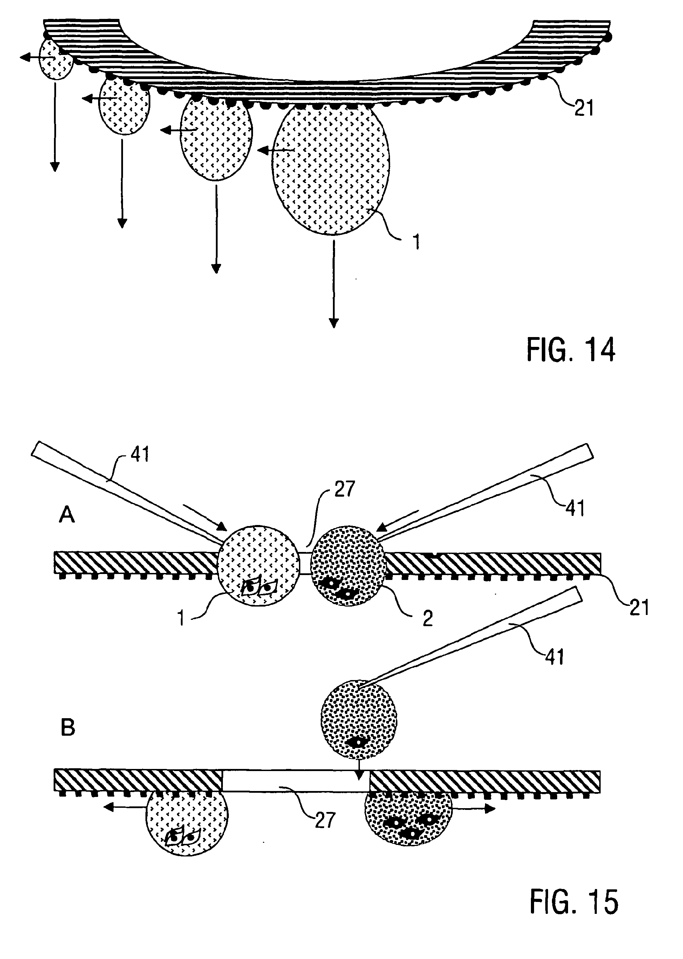

Die Durchgangsöffnung 27 ist z. B. zur Fixierung eines aus zwei Teiltropfen 1, 2 gebildeten verbundenen Tropfens 3 und zur mikroskopischen Beobachtung und/oder Vermessung des verbundenen Tropfens 3 vorgesehen. In einem ersten Teilschritt des erfindungsgemäßen Verfahrens (Figur 10A) werden die Teiltropfen 1, 2 von zwei Seiten zur Durchgangsöffnung 27 bewegt. Gemäß Figur 10B wird auf der Oberseite der Haltefläche 21 eine Messeinrichtung 80, zum Beispiel ein optisches Mikroskop angeordnet, mit dem der verbundene Tropfen 3 in der Durchgangsöffnung 27 beobachtet werden kann. Eine weitere Funktion der Durchgangsöffnung 27 kann in der Erleichterung der Zuführung oder Entnahme von Zellen aus dem verbundenen Tropfen 3 mit einem Zusatzinstrument 40, wie zum Beispiel einer Kapillare (siehe auch Figuren 15 ff.) bestehen.

Figur 11 illustriert weitere Einzelheiten der Kultivierung von Zellen 13 in einem Kultivierungstropfen 7, der an einem feldfreien Teilbereich 25 der Haltefläche 21 hängt (siehe auch Figur 9). Die erfindungsgemäß verwendete Tropfenmanipulationsvorrichtung kann mit einer schematisch gezeigten Kultivierungseinrichtung 70 ausgestattet sein, mit der Kultivierungsbedingungen für die Kultivierung der Zellen 13 einstellbar sind. Die Kultivierungseinrichtung 70 umfasst zum Beispiel eine Temperierungseinrichtung zur Einstellung einer vorbestimmten Kultivierungstemperatur, eine Befeuchtungseinrichtung zur Bereitstellung einer vorbestimmten Umgebungsfeuchte und/oder eine Gaszufuhreinrichtung zur Bildung einer Gasatmosphäre, zum Beispiel CO₂-Atmosphäre um den Kultivierungstropfen 7. In Abhängigkeit von der konkreten Funktion der Kultivierungseinrichtung 70 kann diese mit der Haltefläche 21 verbunden oder unterhalb der Haltefläche 21 angeordnet sein.

Figur 11 zeigt des Weiteren ein Auffangreservoir 50, das zur Aufnahme von Flüssigkeitstropfen von der Haltefläche 21 eingerichtet ist. Das Aufnahmereservoir 50 umfasst zum Beispiel ein Substrat mit einer Vielzahl von Kompartimenten 51 (zum Beispiel eine Mikrotiterplatte oder eine Nanotiterplatte), wobei jedes der Kompartimente zur Aufnahme von einem oder mehreren Kultivierungstropfen vorgesehen ist.

Zur Tropfenablage durch Abtropfen von der Haltefläche 21 wird das Auffangreservoir 50 mit einer Positioniereinrichtung (nicht dargestellt) so unter der Haltefläche 21, insbesondere einer Abtropfposition angeordnet, dass der Kultivierungstropfen 7 in ein vorbestimmtes Kompartiment 51 fällt.

Vorteilhafterweise kann durch ein Verschieben des Auffangreservoirs 50 mit den Kompartimenten 51 eine schnelle und automatisierte Ablage von Tropfen erfolgen, die jeweils eine einzelne Zelle oder eine Zellgruppe 13 enthalten, wie dies für Aufgaben bei der Klonierung oder der Induktion von Gewebewachstum gewünscht ist.

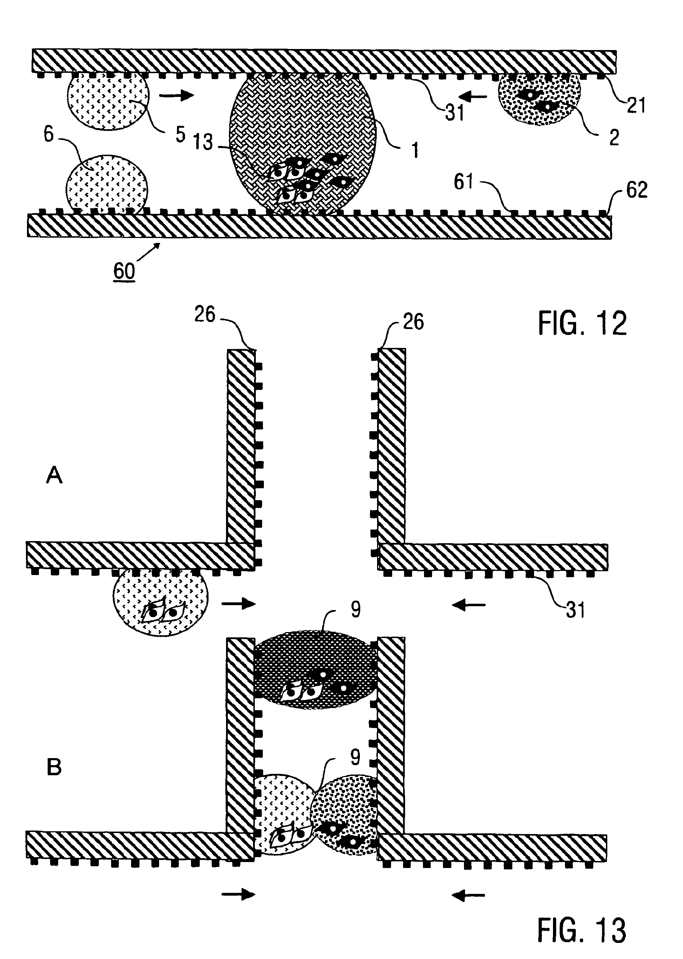

Figur 12 zeigt eine Tropfenmanipulation, bei der die Tropfenmanipulationsvorrichtung mit einem Bodenelement 60 ausgestattet ist, das eine Bodenfläche 61 aufweist. Die Bodenfläche 61 ist ein Bauteil mit einer festen Oberfläche, die zur Unterstützung des Tropfens 1, der an der Haltefläche 21 hängt, und/oder zur Aufnahme eines weiteren Tropfens 6 vorgesehen ist. Auf der Bodenfläche 61 können Elektroden 62 vorgesehen sein, deren Funktion in Abhängigkeit von der konkreten Anwendung der Vorrichtung wählbar ist. Erstens können mindestens zwei Elektroden der Bodenfläche 61 für eine elektrische Messung, zum Beispiel eine Impedanzmessung an den Zellen 13 im Tropfen 1 vorgesehen sein. Des Weiteren können Elektroden 62 an der Position des Tropfens 1 für eine schonende Zellmanipulation mittels negativer Dielektrophorese eingerichtet sein.

Schließlich können die Elektroden 62 auch zur Unterstützung des Tropfentransports verwendet werden, wie er oben unter Bezug auf die erfindungsgemäße Tropfenmanipulation an der Haltefläche 21 beschrieben ist. Beispielsweise kann der Tropfen 6 auf der Bodenfläche 61 unter der Wirkung von Wanderwellen zum Tropfen 1 geschoben werden. Es kann somit auch vorgesehen sein, dass der Tropfen 1 mit mindestens einer Zelle 13 durch elektrische Felder bewegt wird, die sowohl an den Elektroden 31 der Haltefläche 21 als auch an den Elektroden 62 der Bodenfläche 61 erzeugt werden. Zum Tropfentransport sind im Vergleich zum herkömmlichen Tropfentransport mit hochfrequenten elektrischen Feldern geringere Feldstärken erforderlich, so dass eine nachteilige Beeinflussung der mindestens einen Zelle 13 durch Felder in der Nähe der Bodenfläche 61 vermieden werden können.

Der Abstand der Halte- und Bodenflächen 21, 61 kann in Abhängigkeit vom konkret realisierten Verfahren der Tropfenmanipulation gewählt werden. Vorzugsweise ist der Abstand so gewählt (zum Beispiel im Bereich von 50 µm bis 5 mm), dass nur der durch Flüssigkeitszufuhr vergrößerte Tropfen 1 durch die Bodenfläche 61 unterstützt wird, während die Tropfen 2, 5 und 6 zur Zell- und/oder Wirksubstanzzufuhr voneinander getrennt sind. Alternativ kann der Abstand geringer gewählt werden, so dass alle Tropfen gleichzeitig die Halte- und Bodenflächen 21, 61 berühren.

Die Kombination der Halte- und Bodenflächen 21, 61 bietet den Vorteil einer einfachen Beladung der Haltefläche 21 zur Aufnahme hängender Tropfen mit einem herkömmlichen Gerät zur Tropfenbewegung mit hochfrequenten elektrischen Feldern. Beispielsweise kann die Bodenfläche 61 relativ zur Haltefläche 21 versetzt angeordnet sein (nicht dargestellt), um in einem ersten Teilbereich der Bodenfläche 61 Tropfen mit einer Pipette abzulegen und unter der Wirkung von hochfrequenten elektrischen Feldern zu der Haltefläche 21 zu bewegen, wo die Tropfen in den hängenden Zustand überführt und der weiteren Manipulation unterzogen werden. Ein weiterer Vorteil besteht darin, dass mit der Kombination der Halte- und Bodenflächen 21, 61 ein geschlossenes System gebildet und somit die Sterilität der Tropfenmanipulationsvorrichtung leichter sichergestellt werden kann.

Figur 13 illustriert eine weitere Ausführungsform der Erfindung, bei der die Haltefläche 21 mindestens einen vertikalen Teilbereich 26 aufweist. Es kann beispielsweise eine abgewinkelte Haltefläche 21 vorgesehen sein (siehe auch Figur 1C), bei der im vertikalen Teilbereich 26 die Oberflächennormale einen Winkel α = 90° mit der Gravitationsrichtung bildet. Ein einzelner vertikaler Teilbereich 26 kann zum Beispiel für eine Messung am hängenden Tropfen von Vorteil sein.

Besondere Vorteile bietet jedoch die Kombination von zwei vertikalen Teilbereichen 26 (Figur 13A), die einen Spalt bilden. Der senkrechte Abstand der Teilbereiche 26 ist so gewählt, dass mindestens ein Tropfen 9 zwischen den vertikalen Teilbereichen 26 eine Flüssigkeitsbrücke bilden kann (Figur 13B) .

Zur erfindungsgemäßen Tropfenmanipulation können beispielsweise Tropfen mit Zellen 13 verschiedener Zelltypen von entgegengesetzten Seiten zu dem Spalt zwischen den vertikalen Teilbereichen 26 bewegt werden. In diesem Zustand sind die verschiedenen Zelltypen voneinander getrennt. Durch die Verschiebung der Tropfen in den Spalt zwischen den vertikalen Teilbereichen 26 erfolgt eine gegenseitige Anlagerung mit einer Durchmischung, gefolgt von einer Wechselwirkung zwischen den verschiedenen Zelltypen.

Der Abstand der vertikalen Teilbereiche 26 ist beispielsweise im Bereich von 50 µm bis 5 mm gewählt. Vorteilhafterweise können mit der in Figur 13 gezeigten Ausführungsform der Erfindung Tropfen manipuliert werden, die größer als frei hängende Tropfen sind. Es ergeben sich Vorteile insbesondere für die Zellkultivierung, zum Beispiel von höheren tierischen oder pflanzlichen Zellen.

Die Umsetzung der Erfindung ist nicht auf die Verwendung von Halteflächen 21 beschränkt, die vollständig oder in Teilbereichen eben sind. Alternativ kann eine gekrümmte Haltefläche 21 vorgesehen sein (Figur 14). Das illustrierte Beispiel zeichnet sich durch einen von der Mitte zum Rand der Haltefläche 21 sich verringernden Krümmungsradius der Haltefläche 21 aus. Da die kritische Abtropfdimension insbesondere vom Krümmungsradius der Haltefläche 21 abhängt, können vorteilhafterweise mit einer Haltefläche 21 mit verschiedenen Krümmungsradien vorbestimmte Abtropfpositionen für verschieden große Tropfen 1 festgelegt werden.

Eine weitere Variante der Beladung der Haltefläche 21 einer erfindungsgemäß verwendeten Tropfenmanipulationsvorrichtung ist in Figur 15 illustriert. Bei dieser Ausführungsform erfolgt die Beladung an einer Durchgangsöffnung 27 in der Haltefläche 21. Gemäß Figur 15A können Tropfen mit Pipettenspitzen 41 am Rand der Durchgangsöffnung 27 abgelegt werden. Durch Kapillarkräfte werden die Tropfen auf die Unterseite der Haltefläche 21 gezogen und dort weiter im hängenden Zustand manipuliert (Figur 15B).

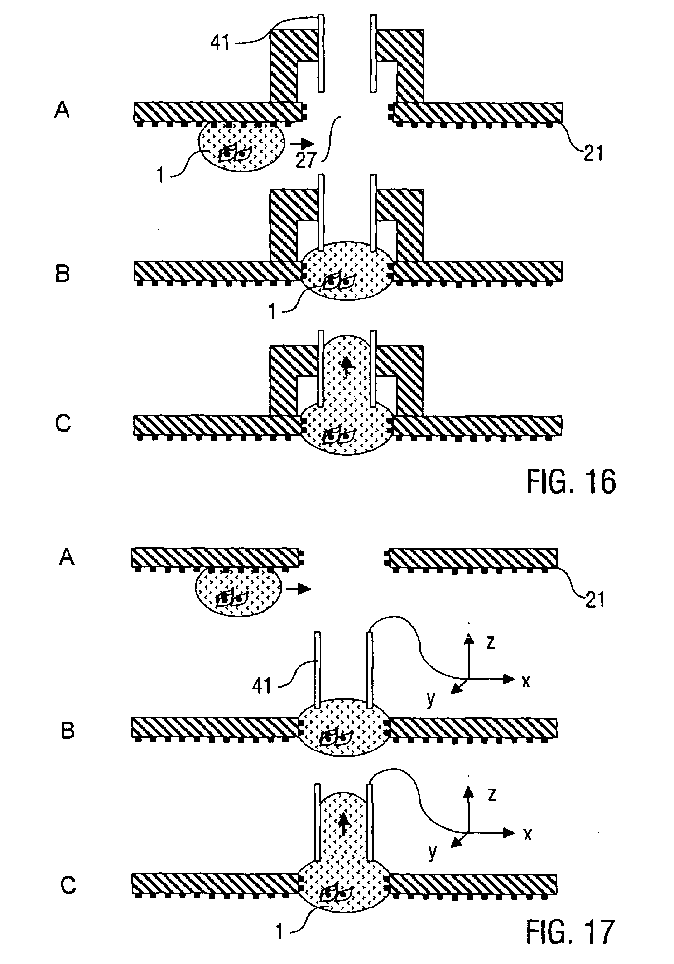

Eine Durchgangsöffnung 27 in der Haltefläche 21 kann zusätzlich oder alternativ zur Tropfenaufnahme von der Haltefläche 21 verwendet werden, wie in den Figuren 15 und 16 illustriert ist. Gemäß Figur 16 ist eine Pipetteneinrichtung 41 (teilweise gezeigt) an der Durchgangsöffnung 27 fixiert. Eine Kapillare oder ein Schlauch der Pipetteneinrichtung 41 endet an oder in der Durchgangsöffnung 27. Zur Aufnahme eines Tropfens 1 von der Haltefläche 21 wird dieser zu der Durchgangsöffnung 27 bewegt (Figur 16A). Zwischen den Rändern der Durchgangsöffnung 27 und dem Ende der Pipetteneinrichtung 41 wird eine Flüssigkeitsbrücke gebildet (Figur 16B). Durch Anlegen eines Unterdrucks an der Pipetteneinrichtung 41 kann der Tropfen abgesaugt werden (Figur 16C).

Zur Zufuhr eines Tropfens zu der Haltefläche 21 können die gezeigten Teilschritte umgekehrt realisiert werden. Ein Tropfen wird mit der Pipetteneinrichtung 41 in der Durchgangsöffnung 27 aufgehängt und durch die Ansteuerung der Elektroden der Haltefläche 21 auf dieser in den hängenden Zustand überführt.

Vorteile für eine erhöhte Flexibilität bei der Zuführung oder Aufnahme von Tropfen zu oder von der Haltefläche 21 ergeben sich, wenn eine bewegliche Pipetteneinrichtung 41 verwendet wird (Figur 17). In diesem Fall ist die Pipetteneinrichtung 41 nicht an der Haltefläche 21 fixiert, sondern in mindestens einer Raumrichtung frei beweglich. An einer Haltefläche 21 können mehrere Durchgangsöffnungen 27 vorgesehen sein. Zur Tropfenaufnahme wird ein Tropfen 1 zu einer freien Durchgangsöffnung 27 bewegt (Figur 17A). Die Pipetteneinrichtung 41 wird ebenfalls zu dieser Durchgangsöffnung 27 bewegt (Figur 17B). Anschließend wird der Tropfen 1 mit der Pipetteneinrichtung 41 abgesaugt (Figur 17C) und zum Beispiel zu einem Auffangreservoir überführt. Zur Tropfenzuführung zur Haltefläche 21 können diese Teilschritte entsprechend mit umgekehrter Richtung realisiert werden.

Der Druck zum Transport des Tropfens in der Pipetteneinrichtung 41 kann durch ein gasförmiges Medium (Figuren 16, 17) oder durch ein flüssiges Medium gebildet werden. Im ersten Fall bleibt der Tropfen in der Pipetteneinrichtung 41 vorteilhafterweise isoliert von anderen Tropfen. Im zweiten Fall kann das flüssige Medium zur Trennung benachbarter Tropfen oder zur Behandlung der im Tropfen enthaltenen Zellen verwendet werden. Die Behandlung kann insbesondere eine Spülung oder Perfusion der Zellen umfassen. Die Perfusion kann bei der Zufuhr oder Aufnahme von Tropfen von der Haltefläche 21 oder in einem Zustand vorgesehen sein, in dem der Tropfen hängend in der Durchgangsöffnung 27 gehalten wird.

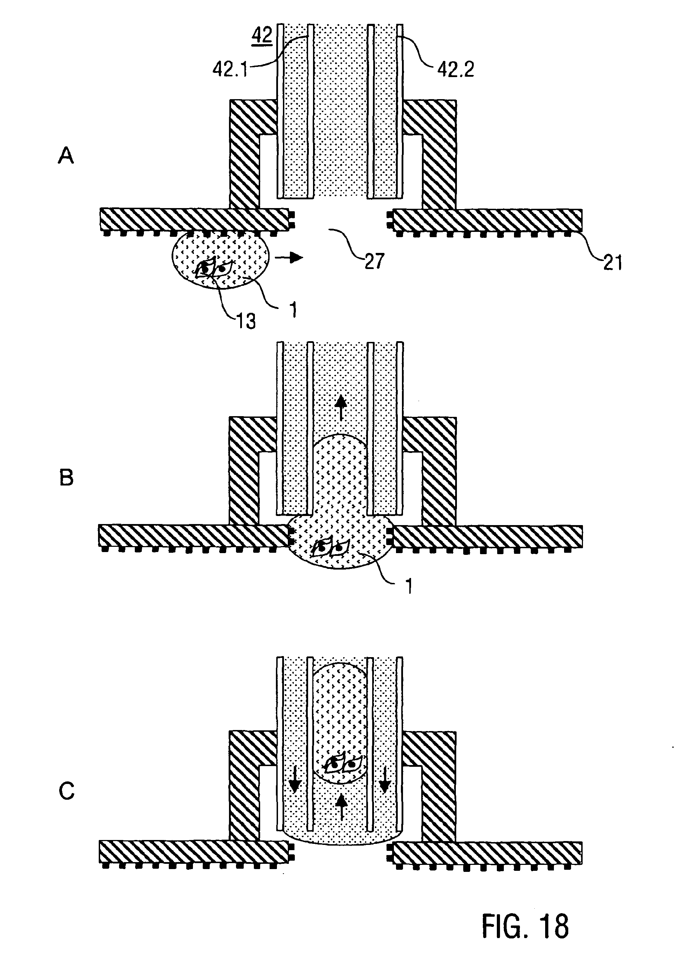

Figur 18 zeigt beispielhaft eine Perfusionseinrichtung 42, die zur Perfusion von Zellen 13 im Tropfen 1 verwendet wird. Die Perfusionseinrichtung 42 umfasst zwei koaxial angeordnete Kapillaren 42.1, 42.2, die an der Durchgangsöffnung 27 fixiert sind.

Zur Perfusion wird in einem ersten Teilschritt (Figur 18A) der Tropfen 1 zur Durchgangsöffnung 27 bewegt und in dieser hängend aufgespannt. Anschließend wird der Tropfen 1 in die innere Kapillare 42.1 eingesaugt (Figur 18B). In einem weiteren Teilschritt (Figur 18C) wird eine Perfusionsflüssigkeit durch die äußere Kapillare 42.2 zugeführt und in die innere Kapillare 42.1 eingesaugt. Dabei wird der Tropfen 1 mit der Perfusionsflüssigkeit durchmischt, so dass die Zellen 13 mit der Perfusionsflüssigkeit umspült werden.

Die Perfusionseinrichtung 42 kann alternativ bei Verwendung einer Transportflüssigkeit, die mit dem Tropfen 1 nicht mischbar ist, zur Aufnahme einer Reihe von Tropfen verwendet werden, welche durch die Transportflüssigkeit voneinander getrennt sind.

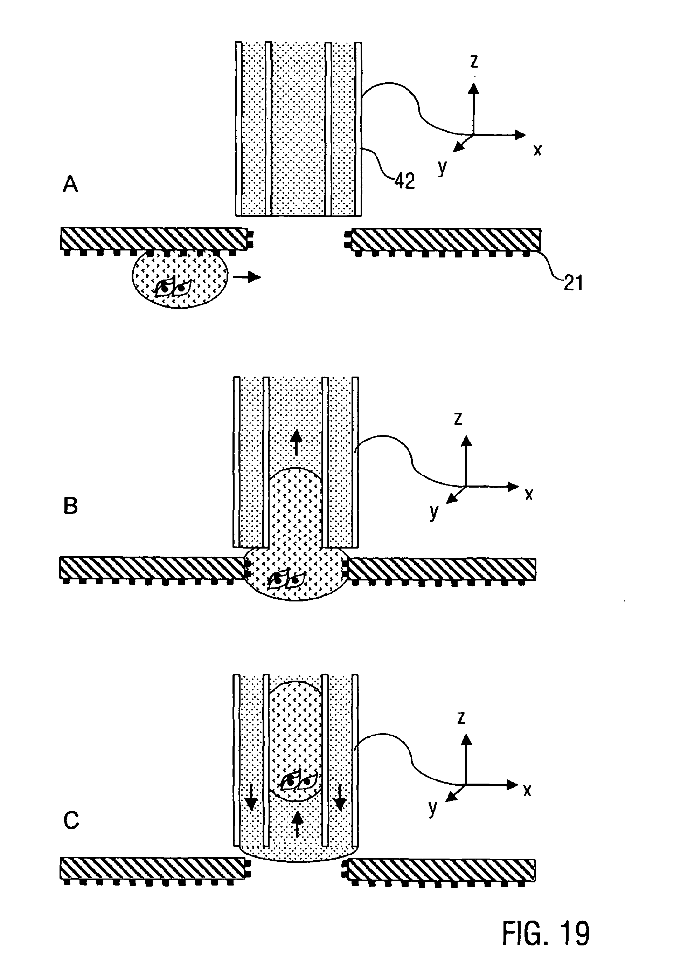

Gemäß einer abgewandelten Ausführungsform der Erfindung kann eine Perfusionseinrichtung 41 vorgesehen sein, die relativ zur Haltefläche 21 beweglich ist. In Figur 19 sind die Teilschritte einer Perfusion oder Tropfenaufnahme mit einer beweglichen Perfusionseinrichtung 41 analog zu Figur 18 gezeigt.

Figur 20 illustriert eine Tropfenmanipulationsvorrichtung, bei der die Elektrodeneinrichtung 30 gemäß der oben genannten zweiten Variante durch eine stab- oder spitzenförmige, bewegliche Elektrode 32 gebildet wird. Im illustrierten Beispiel umfasst die Tropfenhalterung 20 einen Festkörper aus einem inerten Material, zum Beispiel Kunststoff, dessen nach unten weisende Haltefläche 21 zur Aufnahme des hängenden Tropfens 1 vorgesehen ist. Die Tropfenhalterung 20 hat eine Dicke, die einen Durchgriff des elektrischen Feldes von der beweglichen Elektrode 32 zu dem Tropfen 1 ermöglicht. Die Dicke ist vorzugsweise im Bereich unterhalb von 1 mm gewählt. Vorteilhafterweise können einzelne oder mehrere Tropfen 1 sogar an einer Folie aufgehängt sein, welche die Tropfenhalterung 20 bildet.

Die bewegliche Elektrode 32 ist mit einer Spannungsversorgungseinrichtung (nicht dargestellt) und einer Stelleinrichtung (symbolisiert durch das Koordinatensystem) verbunden und auf der Oberseite der Tropfenhalterung 20 beweglich. Die Beweglichkeit ist mindestens in einer Richtung in der X-Y-Ebene entsprechend der Ausrichtung der Haltefläche 21, vorzugsweise in zwei Richtungen in der X-Y-Ebene und besonders bevorzugt in allen drei Raumrichtungen gegeben. Zur Manipulation des Tropfens 1 wird die bewegliche Elektrode 32 mit einer Spannung beaufschlagt, die im Bereich von 20 V bis 500 V gewählt ist. Bei Bewegung der beweglichen Elektrode 32 in der X-Y-Ebene wird der hängende Tropfen 1 auf der Unterseite der Haltefläche 21 mitbewegt.

Figur 20 illustriert des Weiteren, dass der Tropfen 1 auf seiner Unterseite durch eine Bodenfläche 61 unterstützt wird (siehe Figur 12). Auf der Bodenfläche 61 ist eine Flächenelektrode 62 vorgesehen, mit der die Formung des elektrischen Feldes beeinflussbar ist, das auf den Tropfen 1 wirkt. Alternativ kann eine Vielzahl von Elektroden (wie in Figur 12 gezeigt) vorgesehen sein.

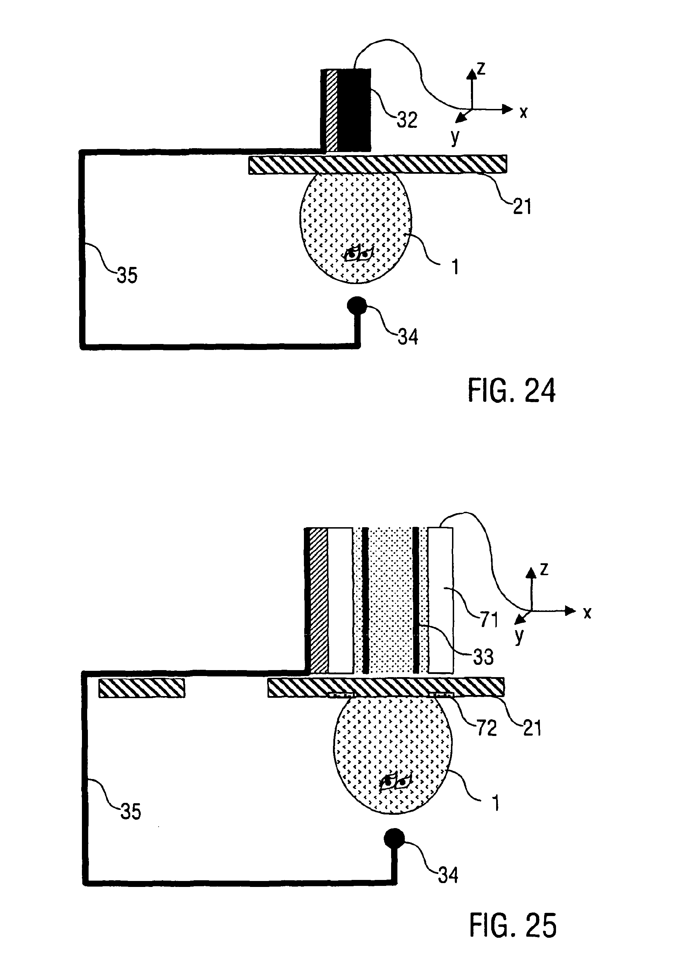

Die Bereitstellung der Bodenfläche 61 mit oder ohne Elektrode 62 ist bei Verwendung der beweglichen Elektrode 32 zur Tropfenmanipulation nicht zwingend erforderlich. Vielmehr kann der Tropfen 1 an der Haltefläche 21 frei hängen (siehe Figur 25) .

Zur Manipulation von mehreren Tropfen, zum Beispiel zur Fusion von Tropfen entsprechend den oben erläuterten Verfahren, kann die Elektrodeneinrichtung 30 zwei oder mehr bewegliche Elektroden 32 aufweisen, die einzeln beweglich und separat elektrisch ansteuerbar sind. Ein Beispiel mit zwei beweglichen Elektroden 32 ist schematisch in Figur 21 gezeigt. Mit den beweglichen Elektroden 32 werden beispielsweise der Tropfen 1 mit Zellen 13 und der Wirksubstanztropfen 6 mit einer Nährstofflösung zueinander geschoben, um einen verbundenen Tropfen zu bilden.

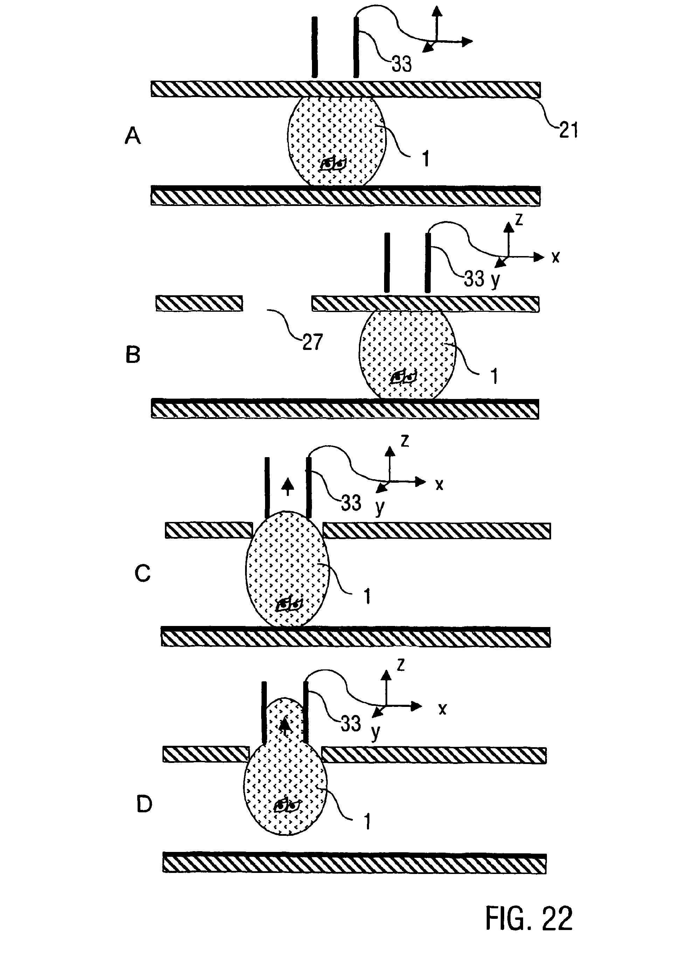

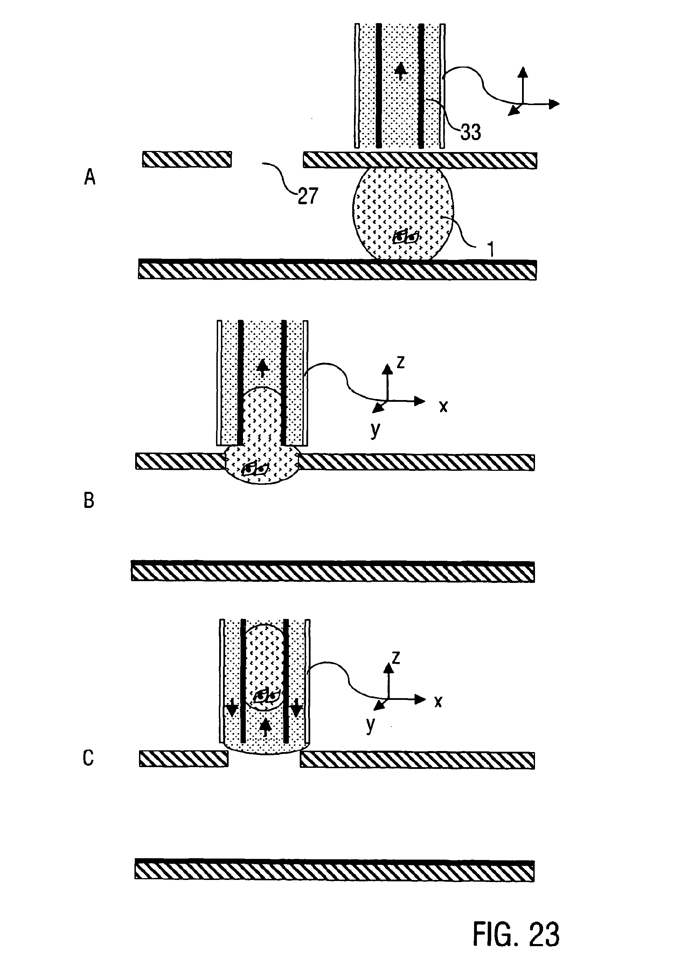

Die Tropfenmanipulationsvorrichtung mit einer beweglichen Elektrode kann vorteilhafterweise verwendet werden, um die oben erläuterten Pipettier- und/oder Perfusionsschritte zu realisieren, wie schematisch in den Figuren 22 und 23 gezeigt ist. Gemäß Figur 22 umfasst die bewegliche Elektrode eine Hohlelektrode 33 mit der Form eines Aufnahmeelements (Spritzennadel, Kanüle, Schlauch oder Kapillare) einer Pipettiereinrichtung. Die Hohlelektrode besteht aus einem elektrisch leitfähigen Material, zum Beispiel aus einem elektrisch leitfähigen Kunststoff, einem Metall oder einem mit Kunststoff beschichtetem Metall. Die Hohlelektrode 33 kann in ersten Teilschritten (Figur 22A, B) verwendet werden, um den Tropfen 1 an der Haltefläche 21 zu einer Durchgangsöffnung 27 zu bewegen. An der Durchgangsöffnung 27 bildet der Tropfen 1 eine Flüssigkeitsbrücke zwischen den Rändern der Durchgangsöffnung 27 und dem unteren Ende der Hohlelektrode 33 (Figur 22C). Bei Beaufschlagung der Hohlelektrode 33 mit einem Unterdruck kann der Tropfen 1 eingesaugt werden (Figur 22D). Die Zufuhr eines Tropfens zur Haltefläche 21 kann mit diesen Teilschritten in umgekehrter Reihenfolge vorgesehen sein.

Die Figuren 23A bis 23C zeigen die Teilschritte der Bewegung des Tropfens 1 zur Durchgangsöffnung 27, des Ansaugens des Tropfens 1 in die Hohlelektrode 33 und die Perfusion des Tropfens (siehe oben, Figur 18). In diesem Fall umfasst die Hohlelektrode 33 eine Koaxialstruktur, von der mindestens eine der inneren und äußeren Kapillare aus einem elektrisch leitfähigen Material gebildet ist.

Die Figuren 24 und 25 zeigen eine weitere Ausführungsform der erfindungsgemäß verwendeten Tropfenmanipulationsvorrichtung, bei der die Elektrodeneinrichtung eine bewegliche Elektrode 32 über der Haltefläche 21 und eine Gegenelektrode 34 unterhalb der Haltefläche 21 und unterhalb des hängenden Tropfens 1 umfasst. Mit der Gegenelektrode 34 kann die Form des Feldes der beweglichen Elektrode 32 und damit die Wirksamkeit der Bewegung des Tropfens 1 an der Haltefläche 21 vorteilhaft beeinflusst werden. In beiden Fällen sind die bewegliche Elektrode 32 und die Gegenelektrode 34 gemeinsam beweglich. Die Bewegung kann mit der Stelleinrichtung der beweglichen Elektrode 32 ausgeführt werden. Hierzu ist die Gegenelektrode 34 über eine die Haltefläche 21 umgreifende Leitungsverbindung 35 mit der beweglichen Elektrode 32 mechanisch verbunden.

Figur 25 zeigt die Kombination einer beweglichen Hohlelektrode 33 mit einer Gegenelektrode 34, wobei zusätzlich als Teil einer Messeinrichtung ein rohrförmiger Lichtleiter 71 zur Beleuchtung der Haltefläche 21 vorgesehen ist. Mit der Beleuchtung können optisch sensitive Funktionsflächen 72 auf der Haltefläche 21 geschaltet und/oder eine optische Messung am Tropfen 1 erleichtert werden. Des Weiteren kann die Kombination der Hohlelektrode 33 und des rohrförmigen Lichtleiters 71 für die obengenannten Funktionen der Zufuhr des Tropfens, Aufnahme des Tropfens oder Perfusion des Tropfens verwendet werden.

Die optisch sensitive Funktionsfläche 72 ist vorzugsweise schicht- und kreisförmig gebildet. Sie besteht zum Beispiel aus einer chemischen Verbindung, die durch Bestrahlung oder Abschattung zwischen einem hydrophilen und einem hydrophoben Zustand umgeschaltet werden kann, wie z. B. Dithienylethen oder Methylorange.

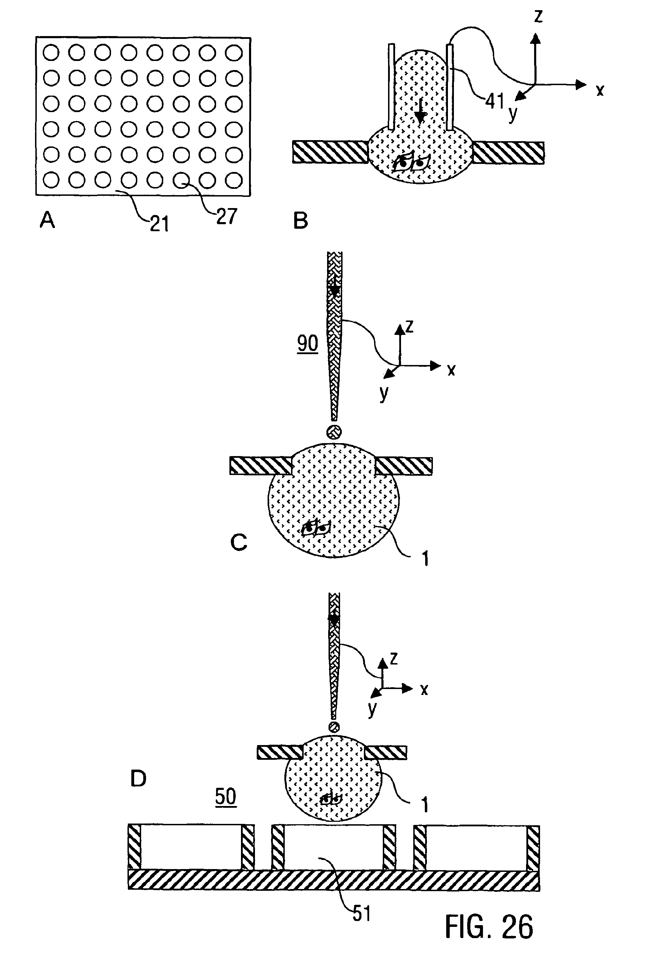

Für die Manipulation einer Vielzahl von Tropfen kann die Tropfenhalterung 20 eine Vielzahl von Durchgangsöffnungen 27 aufweisen, die in der schematischen Draufsicht in Figur 26A gezeigt ist. Über die Durchgangsöffnungen 27 können Tropfen von der Haltefläche 21 aufgenommen oder zu dieser zugeführt werden, wie beispielhaft in den Figuren 26B bis 26D gezeigt ist.

Alternativ zu der Tropfenzufuhr oder -aufnahme mit einer Pipettiereinrichtung 41 (Figur 26B) kann die Tropfenzufuhr mit einer Tropfenzufuhreinrichtung 90 erfolgen (Figur 26C, 26D). Hierzu wird in der Durchgangsöffnung 27 ein Tropfen 1 mit den oben beschriebenen Verfahren aufgespannt. Die Tropfenzufuhreinrichtung 90 umfasst zum Beispiel einen beweglichen Tropfendispenser, mit dem Wirksubstanzen und/oder Zellen dem hängenden Tropfen 1 zugeführt werden. Figur 26C illustriert die Verwendung der Tropfenzufuhreinrichtung 50 zur Vergrößerung des Tropfens oberhalb der Abtropfdimension, so dass eine Ablage in ein Kompartiment 51 des Auffangreservoirs 50 erfolgen kann.

## Patentansprüche

1. Verfahren zur Tropfenmanipulation, mit den Schritten:
- Bereitstellung von mindestens einem Tropfen (1, 2, 3, ...) in Kontakt mit einer festen Haltefläche (21) einer Tropfenhalterung (20),
- Erzeugung eines elektrischen Feldes an der Haltefläche (21), so dass eine Kraft auf den mindestens einen Tropfen (1, 2, 3, ...) ausgeübt wird, und
- Bewegung des mindestens einen Tropfens (1, 2, 3, ...) entlang der Haltefläche unter der Wirkung der Kraft,
**dadurch gekennzeichnet, dass**
- die Haltefläche (21) so ausgerichtet ist, dass eine Oberflächennormale der Haltefläche (21) mit der vertikalen Gravitationsrichtung einen Winkel α kleiner oder gleich 90° bildet, und
- der Tropfen an der Haltefläche (21) hängt.

2. Verfahren gemäß Anspruch 1, bei dem der mindestens eine Tropfen (1, 2, 3, ...) mindestens einen suspendierten Partikel, insbesondere mindestens eine biologische Zelle (13) enthält.

3. Verfahren gemäß Anspruch 1, bei dem der mindestens eine Tropfen (1, 2, 3, ...) frei von suspendierten Partikeln ist.

4. Verfahren gemäß mindestens einem der vorhergehenden Ansprüche, bei dem die Bewegung des mindestens einen Tropfens (1, 2, 3, ...) umfasst:
- eine Verbindung mit mindestens einem weiteren Tropfen (4, 5),
- eine Verschiebung entlang von Teilbereichen (21.1, 21.2) der Haltefläche (21), die in Bezug auf die vertikale Gravitationsrichtung verschieden stark geneigt sind,
- ein Abtropfen an einer Abtropfeinrichtung (23, 25) der Haltefläche (21),
- eine Fixierung an einer Stufenstruktur (24) der Haltefläche (21), und/oder
- eine Verschiebung auf einen feldfreien Teilbereich (25) der Haltefläche (21).

5. Verfahren gemäß Anspruch 4, bei dem die Verbindung mit einem weiteren Tropfen (1, 2, 3, ...) umfasst:
- eine Fusion und Durchmischung von mindestens zwei Tropfen (1, 2), oder
- eine gegenseitige Anlagerung von mindestens zwei Tropfen (1, 4, 5) ohne eine Durchmischung.

6. Verfahren gemäß Anspruch 4 oder 5, bei dem die Verbindung mit einem weiteren Tropfen (1, 2, 3, ...) umfasst:
- eine Vergrößerung der verbundenen Tropfen (1, 4, 5) bis zu einer Größe oberhalb einer kritischen Abtropfdimension und ein Abtropfen von der Haltefläche (21),
- eine Flüssigkeitszufuhr zur Verdunstungskompensation,
- eine Brückenbildung zwischen zwei vertikalen Teilbereichen (26) der Haltefläche (21), und/oder
- eine gegenseitige Wechselwirkung von mindestens zwei Tropfen, die mindestens einen Kultivierungstropfen (1, 7), der mindestens eine biologische Zelle (13) enthält, und/oder mindestens einen Substanztropfen (5, 6) umfassen, der eine Wirksubstanz enthält.

7. Verfahren gemäß mindestens einem der vorhergehenden Ansprüche, mit dem Schritt:
- Kultivierung biologischer Zellen (13) im mindestens einen Tropfen, der an der Haltefläche (21) bewegt wird, oder in einem Kultivierungstropfen (7), zu dem der mindestens eine Tropfen (1, 2, 3, ...) an der Haltefläche (21) bewegt wird.

8. Verfahren gemäß mindestens einem der vorhergehenden Ansprüche, wobei an einer Durchgangsöffnung (27) durch die Haltefläche (21) mindestens einer der Schritte vorgesehen ist:
- Zufuhr des mindestens einen Tropfens (1, 2) zu der Haltefläche (21),
- Fixierung des mindestens einen Tropfens (1, 2),
- Aufnahme des mindestens einen Tropfens (1) von der Haltefläche (21),
- Perfusion des mindestens einen Tropfens (1), und
- Beladung des mindestens einen Tropfens (1).

9. Verfahren gemäß Anspruch 8, mit dem Schritt:
- Abtropfen des mindestens einen Tropfens (1) von der Haltefläche (21).

10. Verfahren gemäß mindestens einem der vorhergehenden Ansprüche, mit dem Schritt:
- Messung mindestens einer physikalischen und/oder chemischen Eigenschaft des mindestens einen Tropfens (1, 2, 3, ...).

11. Kultivierungsgerät für biologische Zellen, umfassend
- eine Tropfenmanipulationsvorrichtung (100), die eine Tropfenhalterung (20) mit einer festen Haltefläche (21), die zur adhäsiven Anordnung von mindestens einem Tropfen (1, 2, 3, ...) eingerichtet ist, und eine Elektrodeneinrichtung (30) umfasst, die zur Erzeugung eines elektrischen Feldes an der Haltefläche (21) eingerichtet ist, so dass eine Kraft auf den mindestens einen Tropfen ausgeübt werden kann, zur Bewegung des mindestens einen Tropfens entlang der Haltefläche, und
- eine Kultivierungseinrichtung (70), die zur Einstellung bestimmter Kultivierungsbedingungen an der Haltefläche (21) vorgesehen ist,
**dadurch gekennzeichnet, dass**
- die Haltefläche (21) zur Aufnahme mindestens eines hängenden Tropfens eingerichtet ist, und
- die Haltefläche (21) im Kultivierungsgerät so ausgerichtet ist, dass eine Oberflächennormale der Haltefläche (21) mit der vertikalen Gravitationsrichtung einen Winkel α kleiner oder gleich 90° bildet.

12. Kultivierungsgerät gemäß Anspruch 11, bei dem die Elektrodeneinrichtung (30) der Tropfenmanipulationsvorrichtung (100) umfasst:
- eine Vielzahl von fixierten Elektroden (31), die auf der Haltefläche (21) angeordnet oder in diese integriert sind, und/oder
- mindestens eine bewegliche Elektrode (32, 33), die auf einer Rückseite (28) der Haltefläche (21) beweglich angeordnet ist.

13. Kultivierungsgerät gemäß Anspruch 11 oder 12, bei dem die Haltefläche (21) der Tropfenmanipulationsvorrichtung (100) umfasst:
- eine Vielzahl von Teilbereichen (21.1, 21.2), die in Bezug auf die vertikale Gravitationsrichtung verschieden stark geneigt sind,
- mindestens einen feldfreien Teilbereich (25) ohne Elektroden (31),
- mindestens zwei vertikal ausgerichtete Teilbereiche (26), zwischen denen der mindestens eine Tropfen (1, 2, 3, ...) eine Brücke (9) bilden kann,
- mindestens eine Abtropfeinrichtung (23, 25), die zum Abtropfen des mindestens einen Tropfens (1, 2, 3, ...) vorgesehen ist,
- mindestens eine Stufenstruktur (24), die zur Fixierung des mindestens einen Tropfens (1, 2, 3, ...) vorgesehen ist, und/oder
- mindestens eine Durchgangsöffnung (27) durch die Haltefläche (21), die zur Zufuhr, Aufnahme, Fixierung, Perfusion und/oder Beladung des mindestens einen Tropfens (1) vorgesehen ist.

14. Kultivierungsgerät gemäß mindestens einem der Ansprüche 11 bis 13, bei dem die Tropfenmanipulationsvorrichtung umfasst:
- eine Pipetteneinrichtung (41),
- eine Perfusionseinrichtung (42),
- eine Tropfenzufuhreinrichtung (43) und/oder
- eine Messeinrichtung (80).

15. Verwendung eines Verfahrens oder eines Kultivierungsgerätes gemäß mindestens einem der vorhergehenden Ansprüche zur Kultivierung und/oder Differenzierung von biologischen Zellen, insbesondere Stammzellen oder Vorläuferzellen.

## Claims

1. A method for the manipulation of drops, comprising the steps of:
- provision of at least one drop (1, 2, 3, ...) in contact with a solid holding surface (21) of a drop holder (20),
- generation of an electric field at the holding surface (21) such that a force is exerted onto the at least one drop (1, 2, 3, ...), and
- movement of the at least one drop (1, 2, 3, ...) along the holding surface under the action of the force,
**characterized in that**
- the holding surface (21) is oriented such that a surface normal of the holding surface (21) forms an angle a with the vertical gravitation direction which is less than or equal to 90°, and
- the drop is hanging from the holding surface (21).

2. The method according to claim 1, in which the at least one drop (1, 2, 3, ...) includes at least one suspended particle, in particular at least one biological cell (13).

3. The method according to claim 1, in which the at least one drop (1, 2, 3, ...) is free from suspended particles.

4. The method according to at least one of the preceding claims, in which the movement of the at least one drop (1, 2, 3, ...) comprises:
- a connection with at least one further drop (4, 5),
- a displacement along subregions (21.1, 21.2) of the holding surface (21), which are inclined to different extents with regard to the vertical gravitation direction,
- a dripping down from a dripping device (23, 25) of the holding surface (21),
- a fixation on a stepped structure (24) of the holding surface (21), and/or
- a displacement onto a field-free subregion (25) of the holding surface (21).

5. The method according to claim 4, in which the connection with one further drop (1, 2, 3, ...) comprises:
- a fusion and thorough mixing of at least two drops (1, 2), or
- a mutual addition of at least two drops (1, 4, 5) without thorough mixing.

6. The method according to claim 4 or 5, in which the connection with one further drop (1, 2, 3, ...) comprises:
- an enlargement of the connected drops (1, 4, 5) up to a size above a critical dripping dimension and a dripping down from the holding surface (21),
- a liquid supply for evaporation compensation,
- a formation of a bridge between two vertical subregions (26) of the holding surface (21), and/or
- a mutual interaction of at least two drops including at least one cultivation drop (1, 7), which includes at least one biological cell (13) and/or at least one substance drop (5, 6), which includes an active substance.

7. The method according to at least one of the preceding claims, comprising the step of:
- cultivation of biological cells (13) in the at least one drop which is moved on the holding surface (21), or in a cultivation drop (7) to which the at least one drop (1, 2, 3, ...) is moved on the holding surface (21).

8. The method according to at least one of the preceding claims, in which at least one of the following steps is provided for at a through-hole (27) through the holding surface (21):
- supply of the at least one drop (1, 2) to the holding surface (21),
- fixation of the at least one drop (1, 2),
- reception of the at least one drop (1) from the holding surface (21),
- perfusion of the at least one drop (1), and
- loading of the at least one drop (1).

9. The method according to claim 8, comprising the step of:
- dripping down of the at least one drop (1) from the holding surface (21).

10. The method according to at least one of the preceding claims, comprising the step of:
- measurement of at least one physical and/or chemical property of the at least one drop (1, 2, 3, ...).

11. A cultivation apparatus for biological cells, comprising:
- a drop manipulation device (100), comprising a drop holder (20) with a solid holding surface (21), which is adapted for an adhesive arrangement of at least one drop (1, 2, 3, ...), and an electrode device (30), which is adapted for the generation of an electric field on the holding surface (21) such that a force can be exerted onto the at least one drop to move the at least one drop along the holding surface, and
- a cultivation device (70) which is provided for the setting of certain cultivation conditions on the holding surface (21),
**characterized in that**
- the holding surface (21) is adapted for the reception of at least one hanging drop, and
- the holding surface (21) is oriented in the cultivation apparatus such that a surface normal of the holding surface (21) forms an angle α with the vertical gravitation direction which is less than or equal to 90°.

12. The cultivation apparatus according to claim 11, in which the electrode device (30) of the drop manipulation device (100) comprises:
- a plurality of fixed electrodes (31) which are arranged on or integrated into the holding surface (21), and/or
- at least one movable electrode (32, 33) which is movably arranged on a rear side (28) of the holding surface (21).

13. The cultivation apparatus according to claim 11 or 12, in which the holding surface (21) of the drop manipulation device (100) comprises:
- a plurality of subregions (21.1, 21.2) which are inclined to different extents with regard to the vertical gravitation direction,
- at least one field-free subregion (25) without electrodes (31),
- at least two vertically oriented subregions (26) between which the at least one drop (1, 2, 3, ...) can form a bridge (9),
- at least one dripping device (23, 25) which is provided for the dripping down of the at least one drop (1, 2, 3, ...),
- at least one stepped structure (24) which is provided for the fixation of the at least one drop (1, 2, 3, ...), and/or
- at least one through-hole (27) through the holding surface (21) which is provided for the supply, reception, fixation, perfusion and/or loading of the at least one drop (1).

14. The cultivation apparatus according to at least one of the claims 11 to 13, in which the drop manipulation device comprises:
- a pipette device (41),
- a perfusion device (42),
- a drop supply device (43), and/or
- a measurement device (80).

15. The use of a method or a cultivation apparatus according to at least one of the preceding claims for the cultivation and/or differentiation of biological cells, in particular stem cells or precursor cells.

## Revendications

1. Procédé de manipulation de gouttes, comportant les étapes suivantes :
- préparation d'au moins une goutte (1, 2, 3, ...) en contact avec une surface de retenue (21) fixe d'un support de gouttes (20),
- génération d'un champ électrique sur la surface de retenue (21), de telle manière qu'une force est exercée sur la ou les gouttes (1, 2, 3, ...), et
- déplacement de la ou des gouttes (1, 2, 3, ...) le long de la surface de retenue sous l'effet de la force,
**caractérisé en ce que**
- la surface de retenue (21) est orientée de telle manière qu'une normale à la surface de retenue (21) forme un angle α inférieur ou égal à 90° avec la direction verticale de gravité, et
- la goutte soit suspendue à la surface de retenue (21).

2. Procédé selon la revendication 1, où la ou les gouttes (1, 2, 3, ...) contiennent au moins une particule en suspension, en particulier au moins une cellule biologique (13).

3. Procédé selon la revendication 1, où la ou les gouttes (1, 2, 3, ...) sont exemptes de particules en suspension.

4. Procédé selon au moins une des revendications précédentes, où le déplacement de la ou des gouttes (1, 2, 3, ...) comprend :
- une liaison avec au moins une autre goutte (4, 5),
- une translation le long de zones partielles (21.1, 21.2) de la surface de retenue (21), présentant des inclinaisons différenciées par rapport à direction de gravité verticale,
- un égouttage contre un dispositif d'égouttage (23, 25) de la surface de retenue (21),
- une fixation contre une structure en gradin (24) de la surface de retenue (21), et/ou
- une translation sur une zone partielle (25) exempte de champ de la surface de retenue (21).

5. Procédé selon la revendication 4, où la liaison avec une autre goutte (1, 2, 3, ...) comprend :
- une fusion et un mélange d'au moins deux gouttes (1, 2), ou
- une adhésion réciproque d'au moins deux gouttes (1, 4, 5) sans mélange.

6. Procédé selon la revendication 4 ou la revendication 5, où la liaison avec une autre goutte (1, 2, 3, ...) comprend :
- un agrandissement des gouttes liées (1, 4, 5) jusqu'à une dimension supérieure à une dimension d'égouttage critique et un égouttage de la surface de retenue (21),
- un apport de liquide pour compensation d'évaporation,
- une formation de pont entre deux zones partielles (26) verticales de la surface de retenue (21), et/ou
- une interaction réciproque d'au moins deux gouttes, qui comprennent au moins une goutte de culture (1, 7) contenant au moins une cellule biologique (13), et/ou au moins une goutte de substance (5, 6) contenant une substance active.

7. Procédé selon au moins une des revendications précédentes, comportant l'étape suivante :
- culture de cellules biologiques (13) dans au moins une goutte déplacée contre la surface de retenue (21), ou dans une goutte de culture (7) vers laquelle la ou les gouttes (1, 2, 3, ...) sont déplacées contre la surface de retenue (21).

8. Procédé selon au moins une des revendications précédentes, où au moins une des étapes suivantes est prévue sur une ouverture de passage (27) au travers de la surface de retenue (21) :
- amenée de la ou des gouttes (1, 2) vers la surface de retenue (21),
- fixation de la ou des gouttes (1, 2),
- réception de la ou des gouttes (1) par la surface de retenue (21),
- perfusion de la ou des gouttes (1), et
- chargement de la ou des gouttes (1).

9. Procédé selon la revendication 8, comportant l'étape suivante :
- égouttage de la ou des gouttes (1) de la surface de retenue (21).

10. Procédé selon au moins une des revendications précédentes, comportant l'étape suivante :
- mesure d'au moins une propriété physique et/ou chimique de la ou des gouttes (1, 2, 3, ...).

11. Appareil de culture pour des cellules biologiques, comprenant :
- un dispositif de manipulation de gouttes (100), comprenant un support de gouttes (20) avec une surface de retenue (21) fixe, prévue pour la disposition par adhérence d'au moins une goutte (1, 2, 3, ...), et un dispositif d'électrode (30), prévu pour la génération d'un champ électrique sur la surface de retenue (21), de telle manière qu'une force peut être exercée sur la ou les gouttes, pour le déplacement de la ou des gouttes le long de la surface de retenue, et
- un dispositif de culture (70), prévu pour l'ajustement de conditions de culture définies sur la surface de retenue (21),
**caractérisé en ce que**
- la surface de retenue (21) est prévue pour la réception d'au moins une goutte suspendue, et
- la surface de retenue (21) est orientée de telle manière dans l'appareil de culture qu'une normale à la surface de retenue (21) forme un angle α inférieur ou égal à 90° avec la direction verticale de gravité.

12. Appareil de culture selon la revendication 11, où le dispositif d'électrode (30) du dispositif de manipulation de gouttes (100) comprend :
- une pluralité d'électrodes fixes (31), disposées sur la surface de retenue (21) ou intégrées à celle-ci, et/ou
- au moins une électrode mobile (32, 33), disposée de manière à être mobile sur une face arrière (28) de la surface de retenue (21).

13. Appareil de culture selon la revendication 11 ou la revendication 12, où la surface de retenue (21) du dispositif de manipulation de gouttes (100) comprend :
- une pluralité de zones partielles (21.1, 21.2), présentant des inclinaisons différenciées par rapport à direction de gravité verticale,
- au moins une zone partielle (25) exempte de champ sans électrodes (31),
- au moins deux zones partielles (26) orientées verticalement, entre lesquelles la ou les gouttes (1, 2, 3, ...) peuvent former un pont (9),
- au moins un dispositif d'égouttage (23, 25), prévu pour l'égouttage de la ou des gouttes (1, 2, 3, ...),
- au moins une structure en gradin (24), prévue pour la fixation de la ou des gouttes (1, 2, 3, ...), et/ou
- au moins une ouverture de passage (27) au travers de la surface de retenue (21), prévue pour l'amenée, la réception, la fixation, la perfusion et/ou le chargement de la ou des gouttes (1).

14. Appareil de culture selon au moins une des revendications 11 à 13, où le dispositif de manipulation de gouttes comprend :
- un dispositif de pipette (41),
- un dispositif de perfusion (42),
- un dispositif d'amenée de gouttes (43) et/ou
- un dispositif de mesure (80).

15. Utilisation d'un procédé ou d'un appareil de culture selon au moins une des revendications précédentes pour la culture et/ou la différenciation de cellules biologiques, en particulier de cellules-souches ou de précurseurs.
